(19) Europäisches Patentamt
European Patent Office
Office européen des brevets

(11) Veröffentlichungsnummer : **0 092 722**
**B1**

(12) # EUROPÄISCHE PATENTSCHRIFT

(45) Veröffentlichungstag der Patentschrift : **10.09.86**

(21) Anmeldenummer : **83103473.1**

(22) Anmeldetag : **11.04.83**

(51) Int. Cl.[4] : **C 07 D501/36**, C 07 D501/20, C 07 D501/32, C 07 D501/57, C 07 D499/70, C 07 D499/68, C 07 D498/04, C 07 D403/12// A61K31/415, A61K31/435, A61K31/535, A61K31/545 ,(C07D498/04, 265:00, 205:00)

(54) **Beta-Lactam-Antibiotika, Verfahren zu ihrer Herstellung sowie ihre Verwendung als Arzneimittel.**

(30) Priorität : **22.04.82 DE 3215085**

(43) Veröffentlichungstag der Anmeldung : **02.11.83 Patentblatt 83/44**

(45) Bekanntmachung des Hinweises auf die Patenterteilung : **10.09.86 Patentblatt 86/37**

(84) Benannte Vertragsstaaten : **CH DE FR GB IT LI**

(56) Entgegenhaltungen :
**EP-A- 0 000 392**
**DE-A- 2 727 586**
**DE-A- 2 941 717**

(73) Patentinhaber : **BAYER AG**
**Konzernverwaltung RP Patentabteilung**
**D-5090 Leverkusen 1 Bayerwerk (DE)**

(72) Erfinder : **Metzger, Karl Georg, Dr.**
**Pahlkestrasse 15**
**D-5600 Wuppertal 1 (DE)**
Erfinder : **Schröck, Wilfried, Dr.**
**Pahlkestrasse 33**
**D-5600 Wuppertal 1 (DE)**
Erfinder : **Häbich, Dieter, Dr.**
**Birkenhöhe 2**
**D-5600 Wuppertal 1 (DE)**
Erfinder : **Naab, Paul, Dr.**
**Schenkstrasse 32b**
**D-5600 Wuppertal 21 (DE)**
Erfinder : **Zeiler, Hans-Joachim, Dr.**
**Elsbeekerstrasse 46**
**D-5620 Velbert 15 (DE)**

**EP 0 092 722 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft β-Lactam-Antibiotika, Verfahren zu ihrer Herstellung sowie ihre Verwendung zur Herstellung von Arzneimitteln und ihre Verwendung zur Förderung des Wachstums und zur Verbesserung der Futterverwertung bei Tieren.

Es ist bereits bekannt geworden, daß bestimmte α-(Imidazolidin-2-oxo-1-yl-carbonylamino)-acetamido-azetidinonderivate, die einen Benzylidenamino-Substituenten am Imidazolidinonring tragen, antibakteriell hoch wirksam sind (DE-OS 3 104 145 $A_1$, DE-OS 2 512 998, DE-OS 2 525 541, DE-OS 2 732 283).

Überraschenderweise zeigen die Azetidinonderivate, die in 3-Stellung einen β-(vic-Dihydroxy-phenylmethylenamino-2-oxo-imidazolidin)- oder β-(vic-Dihydroxy-phenylmethylenamino-2,3-dioxo-piperazin)-1-carbonylamino-acetamido-Rest und in 1-Stellung eine saure Gruppe tragen, eine wesentlich höhere Wirkung gegen Pseudomonasbakterien als die in den obengenannten Offenlegungsschriften beschriebenen und beanspruchten β-Lactame mit Benzylidenamino-imidazolidinon-acetamidosubstituenten.

Die vorliegende Erfindung betrifft daher Verbindungen der Formel I,

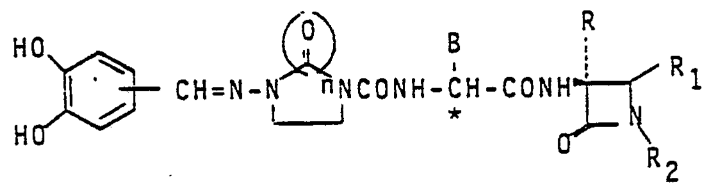

(I)

in welcher

B für gegebenenfalls, durch Methyl, Ethyl, Aminomethyl, Hydroxy, Methoxy, Ethoxy, Carbamoyloxy, Acetoxy, Amino, Mesylamino, Methylamino, Aminosulfonylamino, Guanidyl, Carbamoylamino, Carboxy, Methoxycarbonyl, Carbamoyl, Amidino, Mesyl, Methylsulfinyl, Sulfo, Methylthio oder Halogen einfach oder doppelt substituiertes Phenyl oder Cyclohexadienyl oder für einen ungesättigten gegebenenfalls durch Methyl, Ethyl, Hydroxy, Oxo, Amino, Imino, Mesyl, Mesylamino, Carboxy, Carbamoyl, Acetyl mono- oder disubstituierten 5- oder 6-gliedrigen Heterocyclus mit 1-4 Heteroatomen, die Sauerstoff, Stickstoff oder Schwefel sein können, steht ;

R für Wasserstoff oder Methoxy steht ;

n 1 oder 2 ist ;

$R_1$ Wasserstoff oder gegebenenfalls durch Hydroxy, Amino, Carboxy, Carbamoyl oder Mesyl substituiertes Alkyl mit bis zu 5 C-Atomen, das ungesättigt oder cyclisiert sein kann, ist ;

$R_2$ $SO_3^{\ominus}M^{\oplus}$ ist ;

$M^{\oplus}$ ein Proton oder ein Kation ist ;

oder $R_1$ und $R_2$ zusammen mit dem Azetidinonring, an den sie gebunden sind, für

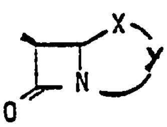

stehen, worin

X für S, O, SO, $SO_2$ oder $CH_2$ steht ; und

Y für die Gruppen

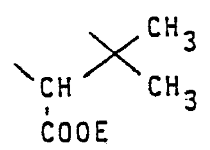

und

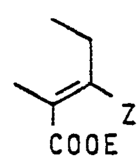

steht,

in welchen das Kohlenstoffatom, das die Gruppe —COOE trägt, an das Stickstoffatom des β-Lactamrings gebunden ist und

Z für Wasserstoff, Halogen, $C_{1-3}$-Alkoxy oder —$CH_2$—T steht,

T Wasserstoff, Acetat, Propionat, n- und i-Butyrat, die durch Carboxy, Hydroxy oder Amino substituiert sein können, Pyridinium, Carboxamidopyridinium, Aminopyridinium, Carbamoyloxy, Azido, Cyano, Hydroxy, die Gruppe —S-Phenyl, welche durch Methyl, Halogen, Amino, Hydroxy oder Carboxy substituiert sein kann, oder die Gruppe —S-Het bedeutet, in welcher Het für einen gegebenenfalls durch Alkyl mit bis zu 3 C-Atomen, welches durch Carboxy, Sulfo, Amino, Methylamino, Dimethylamino oder

Hydroxy substituiert sein kann, durch Hydroxy, Oxo, Amino, Imino oder Sulfo einfach oder zweifach substituierten heterocyclischen 5- oder 6-gliedrigen Ring mit 1-4 Heteroatomen, die Sauerstoff, Stickstoff oder Schwefel sein können, steht ;
und wobei

E für Wasserstoff, eine pharmazeutisch verwendbare Estergruppierung, ein salzbildendes Kation oder eine geeignete Schutzgruppe steht ;
wobei diese Verbindungen der Formel I bezüglich des Chiralitätszentrums $\overset{*}{C}$ in den beiden möglichen R- und S-Konfigurationen sowie als Gemische der daraus resultierenden Diastereomeren vorliegen können, und wobei die Verbindungen der Formel I bezüglich der Iminogruppe sowohl in der syn-Form als auch in der anti-Form vorliegen können und wobei diese Verbindungen der Formel I auch in den verschiedenen Hydratformen vorliegen können.

Für ein Kation $M^{\oplus}$ oder E stehen alle pharmazeutisch verwendbaren Kationen. Als anorganische Kationen können Alkali- und Erdalkalikationen, das Aluminiumkation und das Ammoniumion, als organische Kationen können protonierte organische Amine wie primäre, sekundäre und tertiäre aliphatische Amine sowie heterocyclische Amine eingesetzt werden. Beispielhaft seien genannt : Di- und Triniedrigalkylamine, z. B. Diethylamin, Triethylamin, Aminoethanol, Tri-β-hydroxyethylamin, Procain, Dibenzylamin, N,N'-Dibenzylethylendiamin, N-Benzyl-β-phenyl-ethylamin, N-Methyl- und N-Ethylmorpholin, 1-Ephenamin, Dehydroabiethylamin, N,N'-Bis-dihydroabiethylethylendiamin, N-Niedrigalkylpiperidin. Auch sogenannte basische Aminosäuren wie Lysin oder Arginin können in protonierter Form als Kationen Verwendung finden. Besonders bevorzugt ist das Natriumion.

Als pharmazeutisch verwendbare Estergruppe E kommen diejenigen Estergruppen in Frage, die wie die Pivaloyloximethyl-, die Ethoxycarbonyloxiethyl- oder die Phthalidylgruppe unter physiologischen Bedingungen gespalten werden.

Als geeignete Schutzgruppe E kommen alle in der β-Lactamantibiotikachemie verwendeten Schutzgruppen wie z. B. die Silyl-, Boryl-, Phosphonyl-, Phosphatyl-, Acyl-, Alkoxycarbonyl-, Benzyl-, 2-Acetoacetyl-, t-Butyl-, Benzhydrylgruppe und diejenigen β-substituierten Ethylgruppen in Frage, welche durch nukleophile oder elektrophile Agenzien unter Fragmentierung gespalten werden wie z. B. die β-Silylethyl- oder β-Halogenethylgruppe.

Alle Kristallformen und Hydratformen der erfindungsgemäßen Verbindungen und ihrer Salze sind in gleicher Weise antibakteriell wirksam.

Weiterhin wurde gefunden, daß man die erfindungsgemäßen Verbindungen dadurch erhält, daß man β-(Amino-acetamido)-azetidin-2-one der Formel III

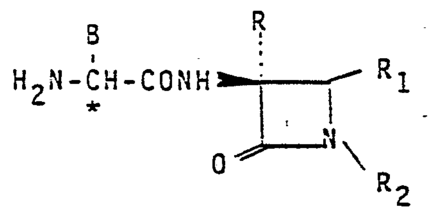
(III)

in der B, R, $R_1$, $R_2$, $M^{\oplus}$, X, Y, Z, T und E wie oben definiert sind,
wobei diese Verbindungen der Formel III bezüglich des Chiralitätszentrums $\overset{*}{C}$ in den beiden möglichen R- und S-Konfigurationen sowie als Gemische der daraus resultierenden Diastereomeren vorliegen können, mit Verbindungen der allgemeinen Formel II

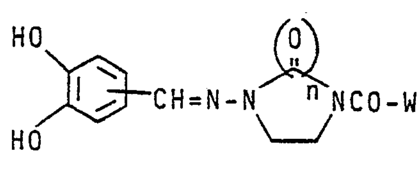
(II)

in welcher

W für Halogen, Azid oder eine andere geeignete nukleofuge Abgangsgruppe steht,

n 1 oder 2 ist und in welcher die beiden Hydroxygruppen durch Silyl- oder Cyclosilylgruppen geschützt sein können, in Gegenwart eines Lösungsmittels und gegebenenfalls eines Säurebindemittels bei Temperaturen von etwa — 20 °C bis etwa + 50 °C umsetzt und die erhaltenen β-Lactam-Antibiotika gegebenenfalls von den vorhandenen Schutzgruppen befreit und gegebenenfalls in ihre pharmazeutisch verwendbaren Salze oder Ester überführt oder aus den erhaltenen Salzen gewünschtenfalls die freien Säuren herstellt.

Bevorzugt sind Verbindungen der Formel I, in welcher

n 1 bedeutet,

$R_2$ $SO_3^{\ominus}M^{\oplus}$ bedeutet, worin $M^{\oplus}$ ein Alkali- oder Erdalkalikation, das Aluminiumkation oder das Ammoniumion, ein protoniertes primäres, sekundäres und tertiäres aliphatisches Amin oder ein heterocyclisches Amin bedeutet,
und solche, in denen

B Phenyl, welches unsubstituiert oder durch Methyl, Ethyl, Aminomethyl, Hydroxy, Methoxy, Ethoxy, Carbamoyloxy, Acetoxy, Amino, Mesylamino, Methylamino, Aminosulfonylamino, Guanidyl, Carbamoylamino, Carboxy, Methoxycarbonyl, Carbamoyl, Amidino, Mesyl, Methylsulfinyl, Sulfo, Methylthio, oder Halogen einfach oder doppelt substituiert ist, oder ein Furyl-, Methylfuryl-, Thienyl, Methylthienyl-, 2-Aminothiazolyl, Thiazolyl-, Methylisoxazoyl-, Isoxazolyl-, Pyridyl-, 2-Aminopyrimidyl-, Thiadiazolyl-, Pyranyl-, Thiapyranyl- oder Sydnonylring ist, und

T Wasserstoff, Acetat, Propionat, n- und i-Butyrat, die durch Carboxy, Hydroxy oder Amino substituiert sein können, Pyridinium, Carboxamidopyridinium, Aminopyridinium, Carbamoyloxy, Azido, Cyano, Hydroxy, die Gruppe —S-Phenyl, welche durch Methyl, Halogen, Amino, Hydroxy, Carboxy substituiert sein kann, oder die Gruppe —S-Het bedeutet, in welcher Het den Thiazol-, Isothiazol-, Thiadiazolyl-, Thiazolyl- oder Tetrazolylring, jeweils unsubstituiert oder durch Methyl, Sulfomethyl, Carboxymethyl, Dimethylaminoethyl substituiert, den Pyridyl-, 1-Oxido-pyridyl-, 2-Methyl-5-oxo-6-hydroxy-1,4-dihydro-1,2,4-Triazin- oder den 4-Formylmethyl-5-oxo-6-hydroxy-4,5-dihydro-1,2,4-triazinylring bedeutet.

Als Verdünnungsmittel kommen beim erfindungsgemäßen Verfahren Wasser sowie alle inerten organischen Lösungsmittel, vorzugsweise solche, welche mit Wasser mischbar sind, in Frage. Hierzu gehören vor allem niedere Dialkylketone, z. B. Aceton, Methylethylketen, cyclische Ether, z. B. Tetrahydrofuran und Dioxan ; Nitrile, z. B. Acetonitril ; niedere Dialkylformamide, z. B. Dimethylformamid ; niedere Alkylalkohole, z. B. Ethanol und Isopropanol sowie Dimethylsulfoxid.

Diese Lösungsmittel können auch in Mischungen untereinander sowie in beliebigen Mischungen einzelner oder mehrerer dieser Lösungsmittel mit Wasser verwendet werden. Das erfindungsgemäße Verfahren kann also durchgeführt werden in Gegenwart von : (a) ausschließlich Wasser, (b) ausschließlich einem oder mehreren organischen Lösungsmitteln oder (c) Wasser und einem oder mehreren organischen Lösungsmitteln. Ist wegen des Vorhandenseins von Wasser eine pH-Messung während der erfindungsgemäßen Reaktion möglich, wird der pH der Reaktionsmischung durch Zusatz von Basen oder durch Verwendung von Puffergemischen vorzugsweise zwischen 6,5 und 7,5 gehalten. Das erfindungsgemäße Verfahren läßt sich aber auch sehr gut in einem anderen pH-Bereich, beispielsweise zwischen 4,5 und 9,0 oder bei pH 2,0 bis 4,5, durchführen. Ferner ist es möglich, die Reaktion in mit Wasser nicht mischbaren Lösungsmitteln, z. B. halogenierten Kohlenwasserstoffen, wie Chloroform oder Methylenchlorid, unter Zusatz von organischen Basen, vorzugsweise Niederalkylaminen, z. B. Triethylamin, Diethylamin oder cyclischen Basen, z. B. N-Ethylpiperidin durchzuführen. Weiterhin läßt sich die Reaktion in einer Mischung aus Wasser und einem mit Wasser nicht mischbaren Lösungsmittel, wie z. B. Niederalkylethern, wie Diethylether, halogenierten Kohlenwasserstoffen, wie Chloroform und Methylenchlorid ; Schwefelkohlenstoff ; Isobutylmethylketon ; Estern wie Essigsäureethylester ; aromatischen Kohlenwasserstoffen wie Benzol, ausführen, wobei es zweckmäßig ist, kräftig zu rühren und den pH-Wert durch Basenzusatz oder Verwendung von üblichen Pufferlösungen, z. B. Phosphat-, Acetat- oder Citratpuffer, zwischen 4,5 und 9,0 oder z. B. 2,0 und 4,5 zu halten. Man kann die Reaktion aber auch in Wasser allein in Abwesenheit von organischen Lösungsmitteln in Gegenwart einer organischen oder anorganischen Base oder unter Zusatz von üblichen Pufferstoffen durchführen.

Als Säurebindemittel können alle in der Chemie der Antibiotika üblicherweise verwendeten Säurebinder verwendet werden. Hierzu gehören anorganische Basen und organische Basen, welche z. B. durch sterische Hinderung schwer acylierbar sind. Als Beispiele für anorganische Basen seien Natrium- und Kaliumhydroxid genannt. Als organische Basen kommen praktisch alle nicht oder schwer acylierbaren offenkettigen oder cyclischen Amine und auch heteroaromatische Basen in Frage. Als Basen seien beispielhaft tertiäre Amine, vorzugsweise Niederalkylamine, z. B. Triethylamin und/oder cyclische Basen, z. B. Pyridin sowie als schwer acylierbares sekundäres Amin Dicyclohexylamin genannt.

Beim erfindungsgemäßen Verfahren ist der Zusatz einer Base nur dann erforderlich, wenn während der Reaktion saure Verbindungen entstehen, z. B. im Falle, daß W für Halogen oder Azid steht.

Die Reaktionstemperaturen können in einem größeren Bereich variiert werden. Im allgemeinen arbeitet man zwischen etwa — 20 °C und etwa + 50 °C, vorzugsweise zwischen 0 und + 20 °C. Wie bei den meisten chemischen Reaktionen können jedoch prinzipiell auch höhere oder niedrigere Temperaturen verwendet werden.

Die Umsetzung kann bei Normaldruck, aber auch bei vermindertem oder erhöhtem Druck ausgeführt werden. Im allgemeinen arbeitet man bei Normaldruck.

Bei der Durchführung des erfindungsgemäßen Verfahrens können die Anteile der 3-(Aminoacetamido)-azetidin-2-one mit einer sauren Gruppierung an $_1$N und der Verbindungen der allgemeinen Formel II in weiten Grenzen variiert werden, ohne daß das Ergebnis nachteilig beeinflußt wird. Die Ausgangsstoffe können z. B. in äquimolekularen Mengen miteinander zur Reaktion gebracht werden. Es kann jedoch zweckmäßig sein, einen der beiden Reaktionspartner im Überschuß zu verwenden, um sich die Reinigung oder Reindarstellung des gewünschten β-Lactam-Antibiotikums zu erleichtern und die Ausbeute zu erhöhen.

Die Menge der gegebenenfalls verwendeten Basen ist z. B. durch die gewünschte Einhaltung eines bestimmten pH-Wertes festgelegt. Wo eine pH-Messung und Einstellung nicht erfolgt oder wegen des Fehlens von ausreichenden Mengen Wasser im Verdünnungsmittel nicht möglich ist oder nicht sinnvoll ist, werden vorzugsweise 2 Moläquivalente Base zugesetzt.

Die Aufarbeitung der Reaktionsansätze zur Herstellung der erfindungsgemäßen Verbindungen und ihrer Salze erfolgt durchweg in der bei diesen Körpern allgemein bekannten Art und Weise. Auch die Isolierung und Reinigung der erfindungsgemäßen Verbindungen sowie die Freisetzung der freien Säuren aus Salzen oder die Umwandlung der freien Säuren in Salze werden nach allgemein üblichen Methoden der organischen Chemie, welche jedem Fachmann geläufig sind, vorgenommen.

Die als Ausgangsstoffe verwendeten 3-(Amino-acetamido)-azetidin-2-one mit einer sauren Gruppierung an $_1$N sind bereits bekannt oder nach bekannten Methoden erhältlich ; alle Kristallformen, Hydratformen und Salze dieser Verbindungen sind als Ausgangsmaterialien für das erfindungsgemäße Verfahren geeignet.

Die als Ausgangsmaterialien verwendeten Verbindungen der allgemeinen Formel II können z. B. auf dem folgenden Weg erhalten werden :

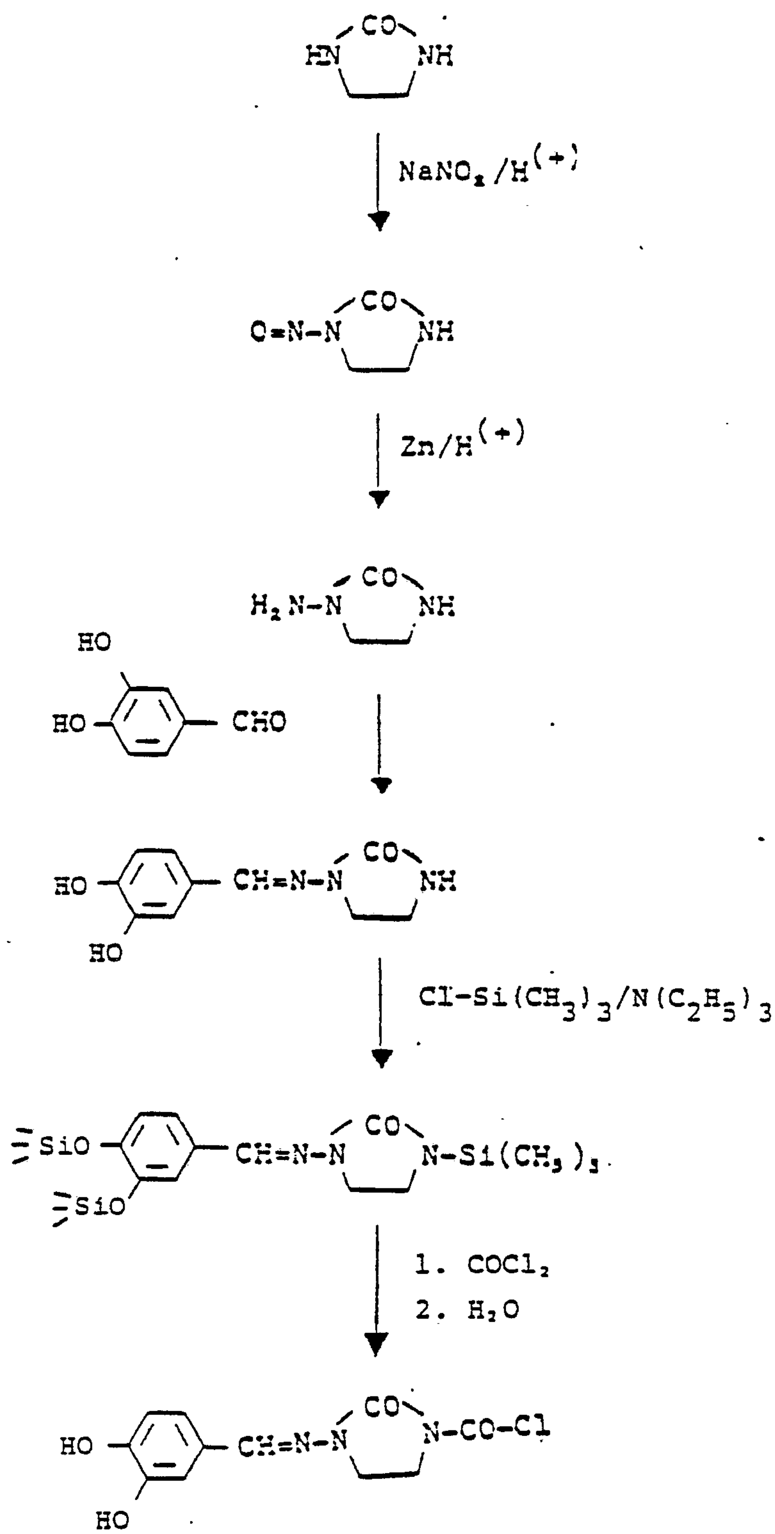

Die Phosgenierung ist auch ohne vorherige Silylierung in einem inerten organischen Lösungsmittel in Gegenwart einer Base möglich. Diejenigen Verbindungen der allgemeinen Formel II, in denen W Azid ist, werden in üblicher Weise z. B. aus den entsprechenden Verbindungen II, in denen W Halogen ist, durch Umsetzung beispielsweise mit Alkaliaziden erhalten.

In einem weiteren erfindungsgemäßen Verfahren werden Verbindungen der allgemeinen Formel IV,

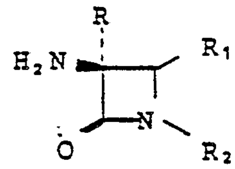

(IV)

in welcher R, $R_1$ und $R_2$ die oben angegebene Bedeutung haben, mit acylierend wirkenden Derivaten einer (vic-Dihydroxyphenylmethylamino)-(2-oxo-imidazolidin-1-yl-) oder (-2,3-dioxo-piperazin-1-yl-) carbonylaminoessigsäure der allgemeinen Formel V,

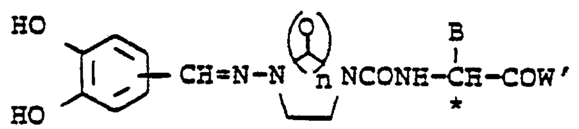

(V)

in welcher n, B und $\underset{*}{C}$ die oben angegebene Bedeutung haben und W' OH oder eine reaktive Abgangsgruppe ist, umgesetzt. Als Verbindung (V) eignen sich z. B. freie Säuren, Säurehalogenide, Säureanhydride, aktive Ester, aktive Amide und Ketene mit dem gewünschten Acylrest. Die Acylierung kann gegebenenfalls in Gegenwart einer Base, wie Triethylamin, Tributylamin, Pyridin oder Natriumhydrogencarbonat, Molekularsieben, Carbodiimiden, z. B. Dicyclohexylcarbodiimid, Epoxiden, z. B. Propylenoxid oder Butylenoxid, oder Enzymen durchgeführt werden. Die Umsetzung kann nach der Säurechlorid-, Säureanhydrid-, Carbodiimid- oder Aktivestermethode erfolgen.

Als Lösungsmittel für diese Reaktionen eignen sich z. B. Ether und cyclische Ether wie Tetrahydrofuran, Ketone wie Aceton, Amide wie Dimethylformamid, chlorierte Kohlenwasserstoffe wie Chloroform, in Gegenwart oder in Abwesenheit von Wasser, bedingt durch die Hydrolyseempfindlichkeit der COW'-Gruppe.

Die als Ausgangsstoffe verwendeten 3-Aminoazetidin-2-one mit einer sauren Gruppierung an $_1$N sind bereits bekannt oder nach bekannten Methoden erhältlich, alle Kristallformen, Hydratformen und Salze dieser Verbindungen sind als Ausgangsmaterialien für das erfindungsgemäße verfahren geeignet.

Die als Ausgangsmaterial verwendeten Verbindungen der allgemeinen Formel V, in denen W' eine reaktive Abgangsgruppe ist, werden aus den Verbindungen der allgemeinen Formel V, in denen W' OH ist, durch Umsetzung mit aktivierenden Mitteln wie anorganischen oder organischen Säurehalogeniden oder -Anhydriden oder durch Kondensation zu aktivierten Estern, Amiden, Hydroxylamiden z. B. mittels Carbodiimiden erhalten.

Die als Ausgangsmaterial verwendeten Verbindungen der allgemeinen Formel V, in denen W' OH ist, werden aus den Verbindungen der allgemeinen Formel II und Aminosäuren erhalten.

Weiterhin wurde gefunden, daß man erfindungsgemäße Verbindungen der allgemeinen Formel VI,

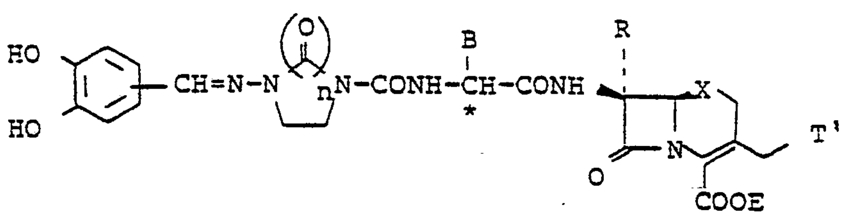

(VI)

in der n, B, $\underset{*}{C}$, R, X und E die oben angegebene Bedeutung haben und T' die oben angegebene Bedeutung von T außer Wasserstoff hat, dadurch erhält, daß man Verbindungen der allgemeinen Formel VII

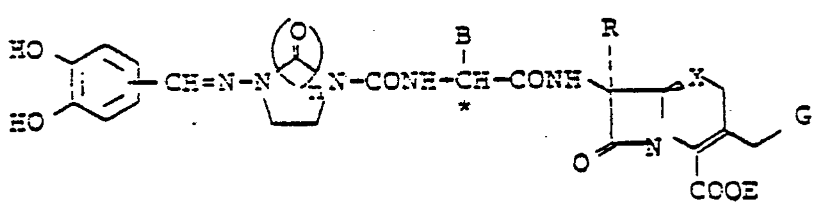

(VII)

in der n, B, $\underset{*}{C}$, R, X und E die oben angegebene Bedeutung haben und G eine nukleofuge Gruppe wie Halogen, Pseudohalogen, Acetoxy, Dichloracetoxy, Mesyloxy bedeutet, mit einem Nukleophil $[T']^-$, in dem T' die oben angegebene Bedeutung hat, unter Austritt von $[G]^-$ zur Umsetzung bringt und gegebenenfalls nach Abspaltung vorhandener Schutzgruppen in die pharmazeutisch verwendbaren Salze oder Ester überführt oder aus den erhaltenen Salzen gewünschtenfalls die freien Säuren herstellt.

Je nach nukleofuger Reaktivität von G sind verschiedene Temperaturen für dieses Verfahren erforderlich ; so lassen sich beispielsweise Verbindungen der allgemeinen Formel VII mit G = Halogen oder Mesyloxy schon bei unter 0° oder auch bei Raumtemperatur umsetzen, während für G z. B. Acetoxy meist Temperaturen über 50 °C bis 120 °C erforderlich sind.

Die als Ausgangsmaterialien für das erfindungsgemäße Verfahren verwendeten Verbindungen der allgemeinen Formel VII werden aus den Verbindungen der allgemeinen Formel V und Verbindungen der allgemeinen Formel XI,

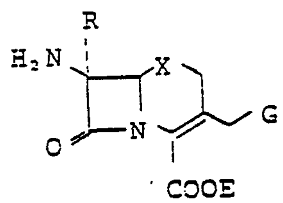

(XI)

in welcher R, E, X und G die oben angegebene Bedeutung haben und die bereits bekannt oder nach bekannten Methoden erhältlich sind, oder aus Verbindungen der allgemeinen Formel II und Verbindungen der allgemeinen Formel XII,

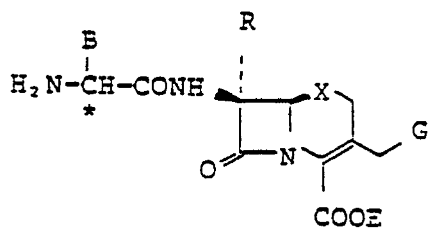

(XII)

in der B, $\underset{*}{C}$, R, X, E und G die oben angegebene Bedeutung haben, oder aus Verbindungen der allgemeinen Formel VII, in denen G OH ist, hergestellt.

Als erfindungsgemäße neue β-Lactam-Antibiotika seien beispielhaft genannt (Formel VIII-X) :

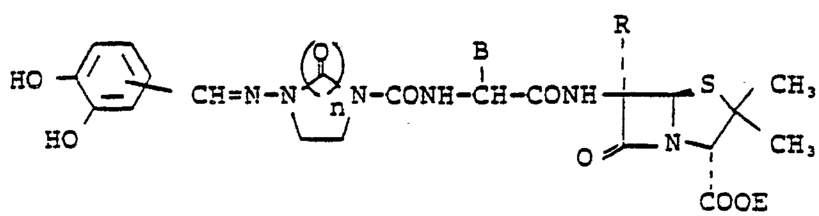

(VIII)

| Stellung der vicinalen OH-Gruppen | n | B | R | E |
|---|---|---|---|---|
| 2,3 | 1 | 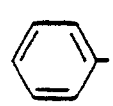 | H | H |
| " | 1 | " | $OCH_3$ | H |
| " | 1 | " | H | $-CH_2OCOC(CH_3)_3$ |

(Fortsetzung)

| Stellung der vicinalen OH-Gruppen | n | B | R | E |
|---|---|---|---|---|
| 3,4 | 1 | " | H | H |
| " | 1 | " | $OCH_3$ | H |
| " | 1 | " | H | $-\underset{CH_3}{CH}-OCOOC_2H_5$ |
| 2,3 | 2 | 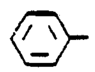 | H | H |
| " | 1 | 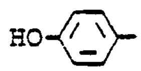 | H | H |
| " | 1 | " | $OCH_3$ | H |
| " | 1 | 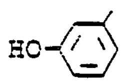 | H | H |
| " | 1 | 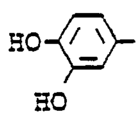 | H | H |
| 3,4 | 1 | 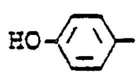 | H | H |
| " | 1 | " | $-OCH_3$ | H |
| " | 1 | 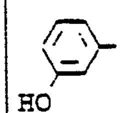 | H | H |
| " | 1 | 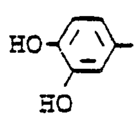 | H | H |
| " | 1 | 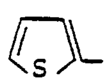 | H | H |
| " | 1 | " | $OCH_3$ | H |
| " | 1 | " | H | 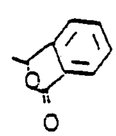 |
| 2,3 | 1 | " | H | H |

(Fortsetzung)

| Stellung der vicinalen OH-Gruppen | n | B | R | E |
|---|---|---|---|---|
| 2,3 | 1 | 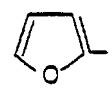 | H | H |
| 3,4 | 1 | " | H | H |
| " | 1 | 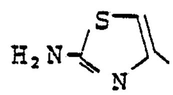 | H | H |
| 2,3 | 1 | " | H | H |
| " | 1 | " | $-OCH_3$ | H |
| " | 1 | 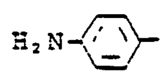 | H | H |
| " | 1 | 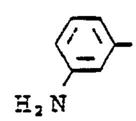 | H | H |
| 3,4 | 1 | " | H | H |
| 3,4 | 1 | 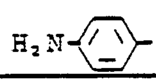 | H | H |

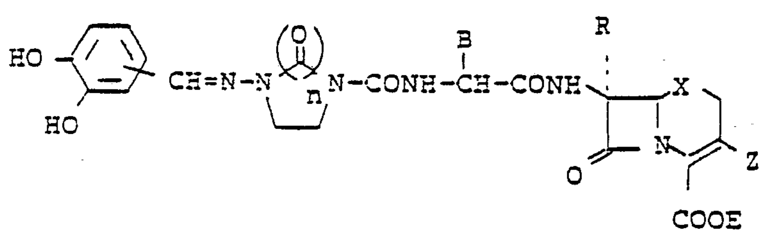

(IX)

(Fortsetzung)

| Stellung der vicinalen OH-Gruppen | n | B | R | X | Z | E |
|---|---|---|---|---|---|---|
| 2,3 | 1 | 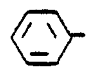 | H | S | $-CH_2OCOCH_3$ | H |
| " | 1 | " | H | S | " | $-CH_2OCO(CH_3)_3$ |
| " | 1 | " | $-OCH_3$ | S | " | H |
| " | 2 | " | H | S | " | H |
| " | 1 | " | H | -SO- | " | H |
| " | 1 | " | H | $-SO_2-$ | " | H |
| " | 1 | " | H | -O- | " | H |
| " | 1 | " | $-OCH_3$ | -O- | " | H |

(Fortsetzung)

| Stellung der vicinalen OH-Gruppen | n | B | R | X | Z | E |
|---|---|---|---|---|---|---|
| 2,3 | 1 | 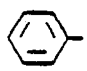 | H | $-CH_2-$ | $-CH_2OCOCH_3$ | H |
| " | 1 | " | H | -S- | 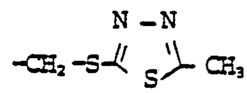 | H |
| " | 1 | " | H | -S- | 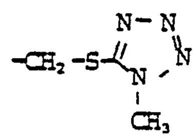 | H |
| " | 1 | " | H | -O- | " | H |
| " | 1 | " | $-OCH_3$ | -O- | " | H |
| " | 1 | " | H | -S- | -Cl | H |
| " | 1 | " | H | -S- | $-OCH_3$ | H |
| " | 1 | " | H | -S- | $-CH_3$ | H |
| " | 1 | " | H | -S- | 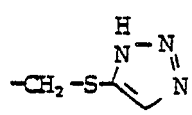 | H |
| " | 1 | " | H | -S- | 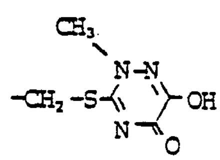 | H |

(Fortsetzung)

| Stellung der vicinalen OH-Gruppen | n | B | R | X | Z | E |
|---|---|---|---|---|---|---|
| 2,3 | 1 | 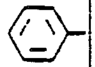 | H | -S- | $-CH_2-S-$ 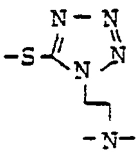 | |
| " | 1 | " | H | -S- | $-CH_2-$ 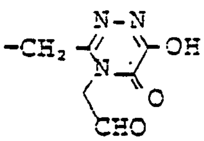 | H |
| " | 1 | " | H | -S- | $-CH_2-$ 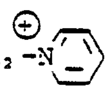 | H |
| " | 1 | " | H | -S- | $-CH_2-$ 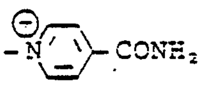 | H |
| " | 1 | " | H | -O- | $-CH_2-S-$ 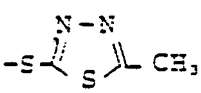 | H |
| " | 1 | " | $-OCH_3$ | -O- | -Cl | H |
| " | 1 | " | H | -O- | $-OCH_3$ | H |
| " | 1 | " | $-OCH_3$ | -O- | $-CH_3$ | H |
| " | 2 | " | H | -O- | $-CH_2-$ 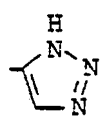 | H |
| " | 1 | " | $-OCH_3$ | -O- | $-CH_2-S-$ 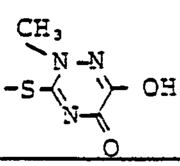 | H |

(Fortsetzung)

| Stellung der vicinalen OH-Gruppen | n | B | R | X | Z | E |
|---|---|---|---|---|---|---|
| 2,3 | 1 | 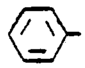 | H | -O- | 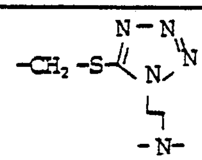 | H |
| " | 1 | " | $-OCH_3$ | -O- | 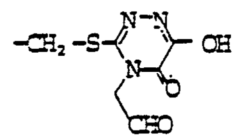 | H |
| " | 1 | " | H | -O- | 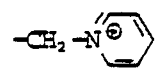 | H |
| " | 1 | " | $-OCH_3$ | -O- | 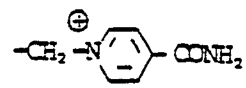 | H |
| " | 1 | " | H | -O- | 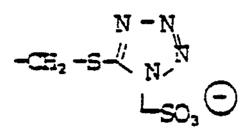 | H |
| " | 1 | " | $-OCH_3$ | -S- | " | H |
| 3,4 | 1 | " | H | S | $-CH_2OCOCH_3$ | H |
| " | 1 | " | H | S | " | $-CH_2OCOC(CH_3)_3$ |
| " | 1 | " | $-OCH_3$ | S | " | H |
| " | 2 | 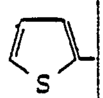 | H | S | " | H |
| " | 1 | 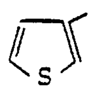 | H | -SO- | " | H |

(Fortsetzung)

| Stellung der vicinalen OH-Gruppen | n | B | R | X | Z | E |
|---|---|---|---|---|---|---|
| 3,4 | 1 | 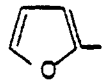 | H | $-SO_2-$ | $-CH_2OCOCH_3$ | H |
| " | 1 | 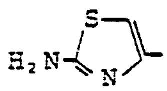 | H | -O- | " | H |
| " | 1 | 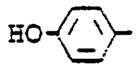 | $-OCH_3$ | -O- | " | H |
| " | 1 | 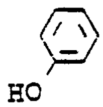 | H | $-CH_2-$ | " | H |
| " | 1 | 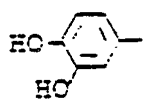 | H | -S- | $-CH_2-S-$ 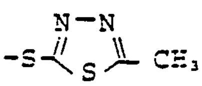 | H |
| " | 1 | 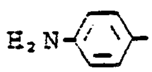 | H | -S- | $-CH_2-S-$ 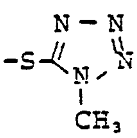 | H |
| " | 1 | 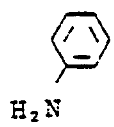 | H | -O- | " | H |
| " | 1 | 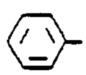 | $-OCH_3$ | -O- | " | H |
| " | 1 | 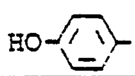 | H | -S- | -Cl | H |

(Fortsetzung)

| Stellung der vicinalen OH-Gruppen | n | B | R | X | Z | E |
|---|---|---|---|---|---|---|
| 3,4 | 1 | 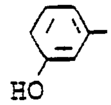 | H | -S- | $-OCH_3$ | H |
| " | 1 | 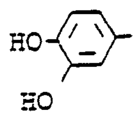 | H | -S- | $-CH_3$ | H |
| " | 1 | 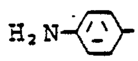 | H | -S- | 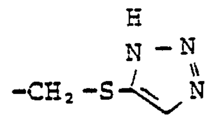 | H |
| " | 1 | 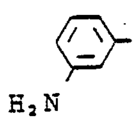 | H | -S- | 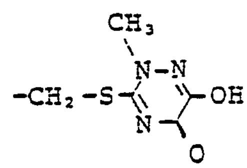 | H |
| " | 1 | 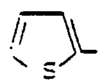 | H | -S- | 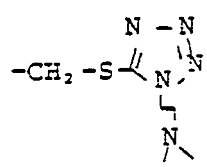 | H |
| " | 1 | 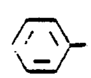 | H | -S- | 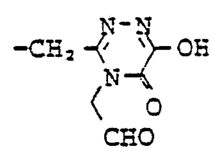 | H |
| " | 1 | 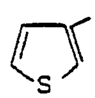 | H | -S- | 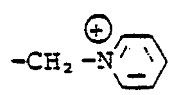 | H |
| " | 1 | 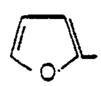 | H | -S- | 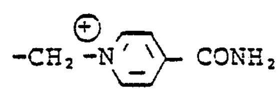 | H |
| " | 1 | 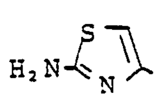 | H | -O- | 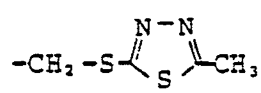 | H |

(Fortsetzung)

| Stellung der vicinalen OH-Gruppen | n | B | R | X | Z | E |
|---|---|---|---|---|---|---|
| 3,4 | 1 | 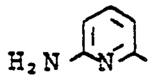 | $-OCH_3$ | -O- | -Cl | H |
| " | 1 | 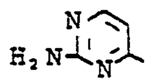 | H | -O- | $-OCH_3$ | H |
| " | 1 | 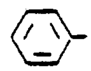 | $-OCH_3$ | -O- | $-CH_3$ | H |
| " | 2 | 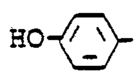 | H | -O- | 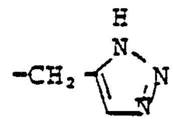 | H |
| " | 1 | 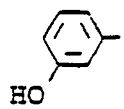 | $-OCH_3$ | -O- | 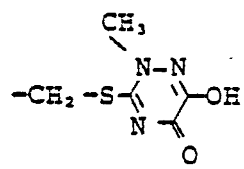 | H |
| " | 1 | 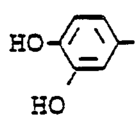 | H | -O- | 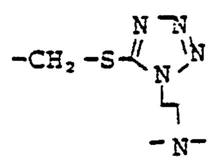 | H |
| " | 1 | 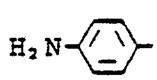 | $-OCH_3$ | -O- | 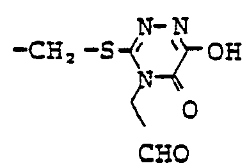 | H |
| " | 1 | 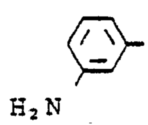 | H | -O- | 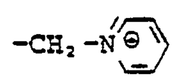 | H |
| 3,4 | 1 | 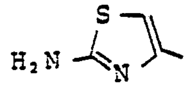 | $-OCH_3$ | -O- | 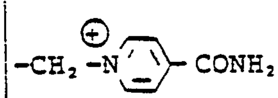 | H |
| " | 1 | 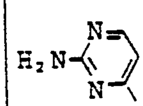 | H | -O- | 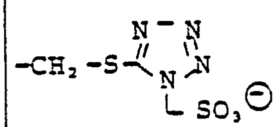 | H |
| " | 1 | 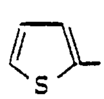 | $-OCH_3$ | -S- | " | H |

(Fortsetzung)

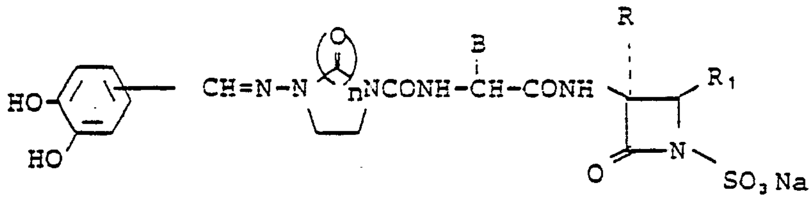

(X)

| Stellung der vicinalen OH-Gruppen | n | B | R | $R_1$ |
|---|---|---|---|---|
| 2,3 | 1 | 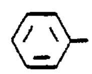 | H | H |
| " | 2 | " | H | H |
| " | 1 | " | $-OCH_3$ | H |
| " | 1 | " | -H | $\alpha$-$CH_3$ |
| " | 1 | " | -H | $\beta$-$CH_3$ |
| " | 1 | " | -H | $\alpha$-$C_2H_5$ |
| " | 1 | " | -H | $\beta$-$C_2H_5$ |
| " | 1 | " | -H | $\alpha$-$CH_2OH$ |
| " | 1 | " | -H | $\beta$-$CH_2OH$ |
| " | 1 | " | -H | $\alpha$-$CH_2COOH$ |
| " | 1 | " | -H | $\beta$-$CH_2COOH$ |
| " | 1 | " | -H | $\alpha$-$CH_2NH_2$ |

(Fortsetzung)

| Stellung der vicinalen OH-Gruppen | n | B | R | $R_1$ |
|---|---|---|---|---|
| 3,4 | 1 | 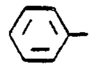 | -H | -H |
| " | 2 | " | -H | -H |
| " | 1 | " | $-OCH_3$ | -H |
| " | 1 | 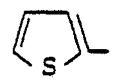 | -H | $\alpha-CH_3$ |
| " | 1 | 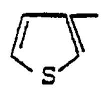 | -H | $\beta-CH_3$ |
| " | 1 | 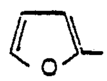 | -H | $\alpha-C_2H_5$ |
| " | 1 | 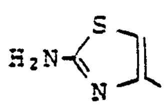 | -H | $\beta-C_2H_5$ |
| " | 1 | 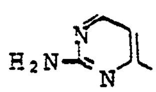 | -H | $\alpha-CH_2OH$ |
| " | 1 | 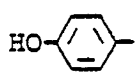 | -H | $\beta-CH_2OH$ |
| " | 1 | 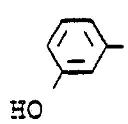 | -H | $\alpha-CH_2COOH$ |
| " | 1 | 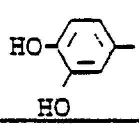 | -H | $\beta-CH_2COOH$ |

(Fortsetzung)

| Stellung der vicinalen OH-Gruppen | B | R | $R_1$ |
|---|---|---|---|
| 3,4 | 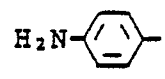 | -H | $\alpha$-$CH_2NH_2$ |
| " | 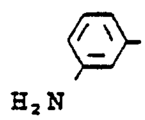 | -H | $\alpha$-$CH_3$ |
| " | H | $-OCH_3$ | H |

Die erfindungsgemäßen Verbindungen sind in Form der freien Säure sowohl kristallin wie amorph und sowohl wasserfrei wie in verschiedenen Hydratformen in gleicher Weise antibakteriell wirksam. Ebenfalls sind diese Verbindungen in Form ihrer Salze, z. B. Natriumsalze, sowohl kristallin wie amorph und sowohl wasserfrei wie wasserhaltig, beispielsweise als Hydrat, in gleicher Weise antibakteriell wirksam.

Die erfindungsgemäßen Verbindungen zeigen neben guter Verträglichkeit und Löslichkeit eine breite antibakterielle Wirkung, d. h. Wirkung gegenüber mehreren Bakterienfamilien im gram-negativen und grampositiven Bereich und gegenüber β-Lactamasebildnern. Aufgrund ihrer starken antibakteriellen Eigenschaften und aufgrund ihrer Fähigkeit, das Wachstum und die Futterverwertung bei Tieren zu verbessern, stellen die erfindungsgemäßen Verbindungen somit eine Bereicherung der Technik dar.

Die erfindungsgemäßen Verbindungen weisen bei geringer Toxizität und guter Verträglichkeit eine starke antimikrobielle Wirksamkeit auf. Diese Eigenschaften ermöglichen ihre Verwendung als Wirkstoffe in der Medizin, wie auch als Stoffe zur Konservierung von anorganischen und organischen Materialien, insbesondere von organischen Materialien aller Art, z. B. Polymeren, Schmiermitteln, Farben, Fasern, Leder, Papier und Holz, von Lebensmitteln und von Wasser.

Die erfindungsgemäßen Wirkstoffe sind gegen ein sehr breites Spektrum von Mikroorganismen wirksam. Mit ihrer Hilfe können z. B. Gram-negative und Gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Wirkstoffe gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemisch Erkrankungen behandelt und/oder verhindert werden, die durch die folgenden Erreger oder durch Mischungen der folgenden Erreger verursacht werden :

Micrococcaceae, wie Staphylokokken, z. B. Staphylococcus aureus, Staph. epidermidis, Staph. aerogenes und Gaffkyatetragena (Staph. = Staphylococcus) ; Lactobacteriaceae, wie Streptokokken, z. B. Streptococcus pyogenes, α- bzw. β-hämolysierende Streptokokken, nicht (γ-)-hämolysierende Streptokokken, Str. viridans, Str. faecalis (Enterokokken), Str. agalactiae, Str. lactis, Str. equi, Str. Anaerobis und Diplococcus pneumoniae (Pneumokokken) (Str. = Streptococcus) ;

Neisseriaceae, wie Neisserien, z. B. Neisseria gonorrhoeae (Gonokokken), N. meningitidis (Meningokokken), N. catarrhalis und N. flava (N. = Neisseria) ;

Corynebacteriaceae, wie Corynebakterien, z. B. Corynebacterium diphtheriae, C. pyogenes, C. diphtheroides, C. acnes, C. parvum, C. bovis, C. renale, C. ovis, C. murisepticum, Listeria-Bakterien, z. B. Listeria monocytogenes, Erysipelothrix-Bakterien, z. B. Erysipelothrix insidiosa ; Kurthia-Bakterien, z. B. Kurthia zopfii (C. = Corynebacterium) ;

Enterobacteriaceae, wie Escherichiae-Bakterien der Coli-Gruppe, Escherichia-Bakterien, z. B. Escherichia coli, Enterobacter-Bakterien, z. B. E. aerogenes , E. cloacae, Klebsiella-Bakterien, z. B. K. pneumoniae, K. ozaenae, Erwiniae, z. B. Erwinia spec., Serratia, z. B. Serratia marcescens (E. = Enterobacter) (K. = Klebsiella), Proteae-Bakterien der Proteus-Gruppe, Proteus, z. B. Proteus vulgaris, Pr. morganii, Pr. rettgeri, Pr. mirabilis (Pr. = Proteus), Providencia z. B. Providencia sp., Salmonelleae, Salmonella-Bakterien, z. B. Salmonella paratyphi A und B, S. typhi, S. enteritidis, S. cholerae suis, S. typhimurium (S. = Salmonella), Shigella-Bakterien, z. B. Shigella dysenteriae, Sh. ambigua, Sh. flexneri, Sh. boydii, Sh. sonnei (Sh. = Shigella) ;

Pseudomonadaceae, wie Pseudomonas-Bakterien, z. B. Pseudomonas aeruginosa, Ps. pseudomallei (Ps. = Pseudomonas) ; Aeromonas-Bakterien, z. B. Aeromonas liquefaciens, A. hydrophile (A. = Aeromonas) ; Spirillaceae, wie Vibrio-Bakterien, z. B. Vibrio cholerae, V. proteus, V. fetus (V. = Vibrio), Spirillum-Bakterien, z. B. Spirillum minus ;

Parvobacteriaceae oder Brucellaceae, wie Pasteurella-Bakterien, z. B. Pasteurella multocida, Past. pestis (Yersinia), Brucella-Bakterien, z. B. Brucella abortus, Br. melitensis, Br. suis (Br. = Brucella), Haemophilus-Bakterien, z. B. Haemophilus influenzae, H. ducreyi, H. suis, H. canis, H. aegypticus (H. = Haemophilus), Bordetella-Bakterien, z. B. Bordetella pertussis, B. bronchiseptica (B. = Bordetella), Moraxella-Bakterien, z. B. Moraxella lacunata ;

Bacterioidaceae, wie Bacteroides-Bakterien, z. B. Bacteroides fragilis, B. serpens (B. = Bacteroides), Fusiforme-Bakterien, z. B. Fusobacterium fusiforme, Sphaerophorus-Bakterien, z. B. Sphaerophorus necrophorus, Sph. necroticus, Sph. pyrogenes (Sph. = Sphaerophorus) ;

Bacillaceae, wie Aerobe Sporenbildner, z. B. Bacillus anthracis, B. subtilis, B. cereus (B. = Bacillus), Anaerobe Sporenbildner-Chlostridien, z. B. Clostridium perfringens, Cl. septicium, Cl. oedematiens, Cl. histolyticum, Cl. tetani, Cl. botulinum (Cl. = Clostridium) ;

Spirochaetaceae, wie Borrelia-Bakterien, z. B. Borrelia recurrentia, B. vincentii (B. = Borrelia), Treponema-Bakterien, z. B. Treponema pallidum., Tr. pertinue, Tr. carateum (Tr. = Treponema), Leptospira-Bakterien, Leptospira interrogans, z. B. Leptospira icterohaemorrhagiae, L. canicola, L. grippotyphosa, L. pomona, L. mitis, L. bovis (L. = Leptospira).

Die obige Aufzählung von Erregern ist lediglich beispielhaft und keineswegs beschränkend aufzufassen.

Ganz besonders wirksam sind die erfindungsgemäßen Wirkstoffe gegen Pseudomonaden, wie aus der folgenden Tabelle hervorgeht :

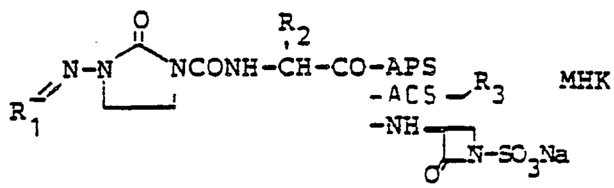

MHK /mcg/ml/

| Verbindung aus Bsp. Nr. | $R_1$ | $R_2$ | | $R_3$ | Psdm.W | Psdm.F41 |
|---|---|---|---|---|---|---|
| 7 c | 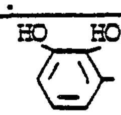 | 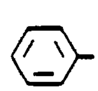 | APS | - | ≤0,25 | ≤0,25 |
| 4 | 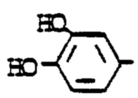 | 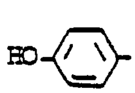 | APS | - | 0,5 | ≤0,25 |
| 14b | " | " | ACS | $-OCOCH_3$ | 0,5 | 0,25 |
| 2d | " | 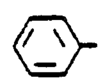 | " | 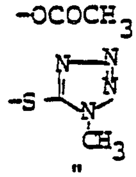 | ≤0,25 | 0,25 |
| 15 | " | 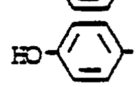 | " | " | 0,25 | ≤0,12 |
| 16b | 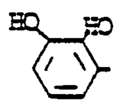 | " | " | $-OCOCH_3$ | 0,25 | 0,25 |
| 17 | " | " | " | 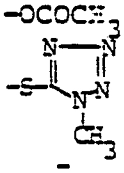 | ≤0,25 | ≤0,25 |
| 23 | 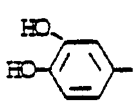 | 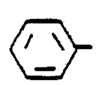 | 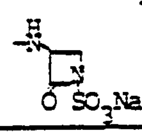 | - | 4 | - |
| - | 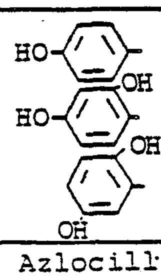 | 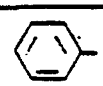 | APS | - | 8 | 8 |
| - | | " | " | - | 64 | 64 |
| - | | " | " | - | 16 | 16 |
| - | Azlocillin | | | | 8 | 8 |
| - | Cefsulodin | | | | 2 | 2 |
| - | Moxalactam | | | | 8-16 | 32 |

Als Krankheiten, die durch die erfindungsgemäßen Wirkstoffe verhindert, gebessert und/oder geheilt werden können, seien beispielhaft genannt:

- Erkrankungen der Atmungswege und des Rachenraumes;

Otitis; Pharyngitis; Pneumonie; Peritonitis; Pyelonephritis; Cystitis; Endocarditis; Systeminfektionen; Bronchitis; Arthritis.

Zur vorliegenden Erfindung gehören pharmazeutische Zubereitungen, die neben nichttoxischen, inerten pharmazeutisch geeigneten Trägerstoffen einen oder mehrere erfindungsgemäße Wirkstoffe enthalten oder die aus einem oder mehreren erfindungsgemäßen Wirkstoffen bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entspricht. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumbicarbonat, (e) Lösungsverzögerer, z. B. Paraffin und (f) Resorptionsbeschleuniger, z. B. quarternäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben anliegenden Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z. B. Kakaofett und höhere Ester (z. B. $C_{14}$-Alkohol mit $C_{16}$-Fettsäure) und Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyethylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel z. B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z. B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Ethylalkohol, Propylenglykol, Suspendiermittel, z. B. ethoxylierte Isostearylalkohole, Polyoxyethylensorbit- und Sorbitanester, mikrokristalline Cellulose, Aluminiummetahydroxid, Bentonit, Agar-Agar und Tragant oder Gemische der Stoffe enthalten.

Die genannten Formulierungsformen können auch Färbemittel, Konservierungsstoffe sowie geruchs- und geschmacksverbessernde Zusätze, z. B. Pfefferminzöl und Eukalyptusöl und Süßmittel, z. B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gewichtsprozent der Gesamtmischung vorhanden sein.

Die oben aufgeführten pharmazeutischen Zubereitungen können außer den erfindungsgemäßen

Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung der oben aufgeführten Erkrankungen.

Die Wirkstoffe oder die pharmazeutischen Zubereitungen können lokal, oral, parenteral, intraperitoneal und/oder rectal, vorzugsweise parenteral, insbesondere intravenös und intramusculär appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 6 bis etwa 800, vorzugsweise 15 bis 300 mg/kg Körpergewicht je 24 Stunden, gegebenenfalls in Form mehrerer, z. B. von 3 Einzelgaben zur Erzielung der gewünschten Ergebnisse zu verabreichen. Die Einzelgabe enthält den oder die erfindungsgemäßen Wirkstoffe, vorzugsweise in Mengen von etwa 2 bis etwa 300, insbesondere 10 bis 150 mg/kg Körpergewicht. Es kann jedoch erforderlich sein, von den genannten Dosierungen abzuweichen und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Im Falle der Anwendung als Futterzusatzmittel können die neuen Verbindungen in üblicher Weise zusammen mit dem Futter bzw. mit Futterzubereitungen oder mit dem Trinkwasser gegeben werden. Dadurch kann eine Infektion durch gram-negative oder gram-positive Bakterien verhindert und ebenso eine bessere Verwertung des Futters erreicht werden.

Die neuen β-Lactam-Antibiotika zeichnen sich durch starke antibakterielle Wirkungen, die *in vivo* und *in vitro* geprüft wurden und durch orale Resorbierbarkeit aus.

Die erfindungsgemäßen β-Lactam-Antibiotika können zum Zwecke der Erweiterung des Wirkungsspektrums oder zur Wirkungssteigerung z. B. auch mit Aminoglykosidantibiotika wie Gentamicin, Sisomicin, Kanamicin, Amikacin oder Tobramicin sowie mit β-Lactamaseinhibitoren wie Clavulansäure, Penicillansäure-S-dioxid oder Olivansäuren kombiniert werden.

## Experimenteller Teil

### Beispiel 1

1a. 1-(3,4-Dihydroxy-benzylidenamino)-2-oxo-imidazolidin

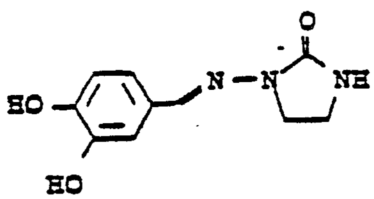

27,6 g (0,2 Mol) 3,4-Dihydroxybenzaldehyd in 100 ml 80 %igem Alkohol und 21,2 g (0,208 Mol) Aminoimidazolidinon in 100 ml des gleichen Lösungsmittels wurden vereinigt. Nach wenigen Sekunden begann Kristallisation des Produktes, das nach 20 Minuten abgesaugt, mit 100 ml des gleichen Lösungsmittels gewaschen und im Exsiccator über $P_2O_5$ getrocknet wurde.

Ausbeute 42,3 g (95,7 %). Fp. 260 °C.

| | | | | |
|---|---|---|---|---|
| Ber. | C 54,3 | H 5,0 | N 19,0 | O 21,7 |
| Gef. | C 54,1 | H 5,0 | N 18,9 | O 21,5 |

IR-Banden bei 1 687, 1 607, 1 295, 1 278, 1 249, 1 230, 1 200, 1 118, 921, 838, 753, 718 $cm^{-1}$.

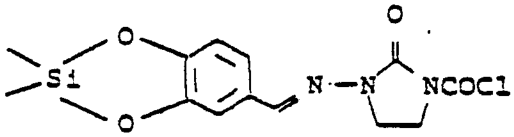

1 b.

51,3 g (0,23 Mol) Produkt aus Beispiel 1a in 500 ml Essigester wurden mit 36,2 g (0,25 Mol) Dimethyldichlorsilan und 140 g (0,756 Mol) Tributylamin versetzt und 30 Min. bei 40-50 °C, dann noch kurz bei 65 °C gehalten,. bis weitgehende Lösung eintrat. Man kühlt auf ca. 5 °C und versetzt mit flüssigem Phosgen (16,8 ml, 0,23 Mol) wobei die Innentemperatur auf 28 °C stieg. Man ließ nun über Nacht rühren, saugte den ausgefallenen Niederschlag ab, wusch mit Essigester und Ether nach und trocknete im Exsiccator.

$Fp_{(Zers.)}$ = 200 °C. Ausbeute 64,7 g (82 %).

| | | | | |
|---|---|---|---|---|
| ber. : | C 46,0 | H 4,1 | Cl 10,4 | N 12,4 |
| gef. : | C 46,5 | H 4,5 | Cl 10,4 | N 12,0 |

IR-Banden bei 1 800, 1 725, 1 255, 1 203, 1 160, 1 102, 953, 900, 814, 730 $cm^{-1}$.

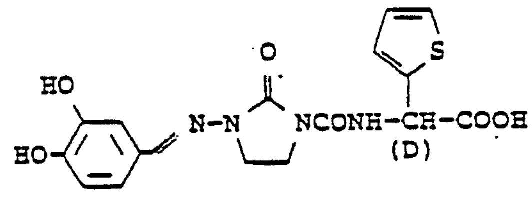

1 c.

30 mM (4,7 g) D-α-(2-Thienyl)-glycin, 70 mM (7,7 g) Trimethylchlorsilan, 70 mM (9,9 ml) Triethylamin und 70 ml Tetrahydrofuran wurden zwei Stunden zum Sieden erhitzt und unter Feuchtigkeitsausschluß abgesaugt. Unter Eiskühlung wurde das Säurechlorid des Beispiels 1b (30 mM) eingetragen und 2 Std. mit der Mischung gerührt. Man fügte 50 ml Wasser zu, stelle pH 7 ein, zog THF im Vakuum ab, extrahierte 1 mal mit Essigester und säuerte die wäßrige Phase auf pH = 2 mit 1 n HCl an, nachdem von 1,8 g eines Niederschlages abgesaugt worden war. Der Niederschlag wurde abgesaugt, mit Wasser gewaschen und im Exsicc. getrocknet.

Ausbeute 10,6 g (90 %).

$Fp._{(Zers.)}$ 210-240 °C.

Berechnet für 98 % + 2 % $H_2O$ :

| | | | | |
|---|---|---|---|---|
| | C 49,5 | H 4,1 | N 13,6 | S 7,7 |
| gef. : | C 49,3 | H 4,3 | N 13,4 | S 7,6 |

IR-Banden bei 3 500-2 500, 1 715, 1 650, 1 540, 1 275, 1 212 $cm^{-1}$.

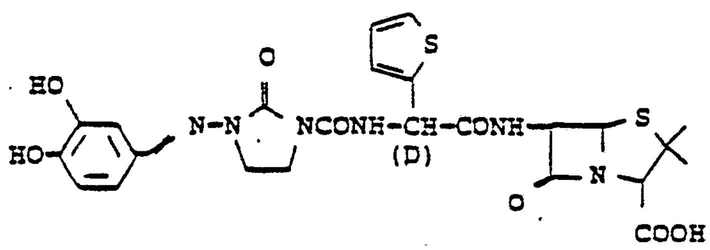

1 d.

3,9 g (19 mM) Präcursorsäure von Beispiel 1c in 40 ml DMF wurden mit 1,01 g (10 mM) N-Methylmorpholin 10 Min. gerührt auf —40 °C abgekühlt. Nun fügte man 1,02 ml (10,6 mM) Chlorameisensäure hinzu, ließ auf —30 °C kommen, rührte 10 Min. bei dieser Temperatur und kühlte wieder auf —55°. Eine inzwischen bereitete und auf 0° gekühlte Lösung aus 2,2 g (10 mM) 6-Aminopenicillansäure in 10 ml Wasser (pH 7,8 mit 1 n NaOH eingestellt) wurde unter heftigem Rühren in einem Guß zugegeben, wobei die Temperatur auf —20 bis —30 °C stieg. Man ließ auf 0 °C kommen und goß auf 500 ml Wasser, stellte pH = 7 ein, saugte von Ungelöstem ab (hier nur ganz geringe Menge) und säuerte mit 1 n HCl auf pH = 2 an. Es wurde abgesaugt, in 300 ml Wasser mit 2 n NaOH wieder auf pH 7,5 gestellt (Lösung) und erneut mit 1 n HCl auf pH = 2 angesäuert. Das Produkt wurde abgesaugt, mit Wasser gewaschen und im Exsiccator über $P_2O_5$ getrocknet.

Ausbeute 5,3 g.

IR-Banden bei 3 600-2 500, 1 768, 1 716, 1 663, 1 512, 1 277, 1 217 $cm^{-1}$.

NMR-Signale bei τ = 0,7 (2H), 2,2 (1H), 2,45 (1H), 2,6 (1H), 2,74 (1H), 2,9 (2H), 3,1 (1H), 3,8 (1H), 4,2 (1H), 4,4 (1H), 5,65 (1H), 6,1 (4H), 8,35 (3H) und 8,47 ppm (3H).

Beispiel 2

2a. 1-Chlorcarbonyl-2-oxo-3-(3,4-dihydroxybenzylidenamino)-imidazolidinon

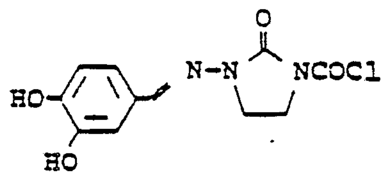

8 g (36 mM) Produkt von Versuch 1a wurden unter Feuchtigkeitsausschluß in 100 ml Essigester unter Eiskühlung mit 8,6 g (79 mM) Trimethylchlorsilan und anschließend mit 21,3 g (115 mM) Tributylamin versetzt und dann zum Rückfluß erhitzt wobei schnell alles in Lösung ging. Es wurde nun auf 0° gekühlt, mit 3,6 g = 2,6 ml (36 mM) flüssigem Phosgen versetzt und zunächst bei 0 °C, dann bei Raumtemperatur über Nacht stehen gelassen. Man versetzte nun unter kräftigem Umschwenken mit ca. 60 g Eis und saugte nach 10 Minuten den entstandenen Niederschlag ab, wusch mit Eiswasser, dann mit Essigester aus und trocknete im Exsiccator über $P_2O_5$.10,3 g. Fp ~ 145 °C. Dieses Produkt (5 g) wurde mit 80 ml Aceton aufgekocht. Der unlösliche Niederschlag wurde abgesaugt und getrocknet. Fr. 1-1. 2 g. $Fp_{Zers.}$ = 220 °C. Die Mutterlauge wurde mit ~ 100 ml Ether gefällt, bei 0 °C stehen gelassen, abgesaugt, mit Ether gewaschen und im Vakuum getrocknet. Fr. 1-2. 1,2 g. Fp. ~ 142°.

Frakt. 1-1 : ber. : C 46,6 H 3,6 Cl 12,5 N 14,8 O 22,6
C 47,1 H 4,0 Cl 11,3 N 14,3 O 22,3

IR-Banden bei 3 240, 1 780, 1 705, 1 590, 1 283, 1 260, 1 198, 1 160, 1 107, 959 und 798 $cm^{-1}$.

Frakt. 1-2 : berechnet für 55 % Produkt + 45 % Tributylhydro-
chlorid : C 54,9 H 7,7 Cl 14,1 N 11,0 O 12,4
gef : C 54,9 H 7,4 Cl 13,7 N 10,8 O 12,2

IR-Banden bei 3 250, 3 125, 2 680, 2 640, 1 800, 1 743, 1 598, 1 278, 1 200, 1 163, 1 105 und 728 $cm^{-1}$.

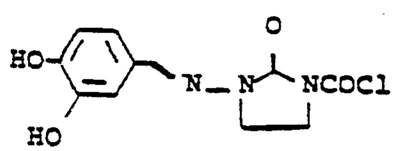

2 b.

6 g (27 mM) Produkt von Versuch 1a wurden bei 0 °C unter Feuchtigkeitsausschluß in 54 ml Essigester suspendiert, mit 2,2 ml (0,03 M) Phosgen und 6 g (0,03 M) Tri-n-butylamin versetzt und bei Raumtemperatur 3 Stdn. gerührt. Es wurde abgesaugt, mit Essigester gewaschen und getrocknet.
Ausbeute 5,8 g.
Die Analyse entsprach 77,5 % Produkt + 21,2 % $Bu_3N$ + 1,3 % Rest.

ber. : C 51,9 H 5,7 Cl 9,7 N 13.0
gef. : C 51,9 H 5,5 Cl 9,7 N 13,5

2c. D-α-[2-Oxo-(3,4-dihydroxy-benzylidenamino)-imidazolidin-1-yl-carbonylamino]-phenylessigsäure

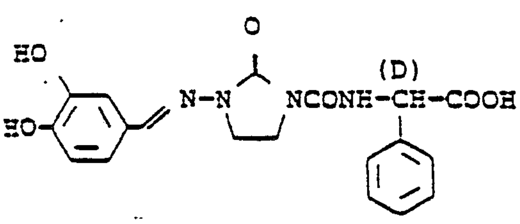

20 mM (5,7 g) Säurechlorid von Versuch 2a. wurden zur Suspension von 20 mM (3,02 g) D-Phenylglycin in 30 ml THF/$H_2O$ (1 : 1) gefügt und mit 2 n NaOH während 1 Std. auf pH = 8 gehalten. Anschließend erwärmte man zur Vervollständigung der Reaktion noch 30 Min. auf 40-50 °C bei pH = 8 und weitere 30 Min. auf 70 °C. Nun kühlte man ab, filtrierte von 0,2 g unverändertem Säurechlorid und säuerte das Filtrat auf pH = 2 mit 5 n HCl an. Es wurde abgesaugt, mit Wasser gewaschen und getrocknet. 4,2 g.

Berechnet für 77 % Produkt, 21 % 1-(3,4-Dihydroxybenzylidenamino)-2-oxo-imidazolidin + 2 % $H_2O$ :

C 55,5 H 4,8 N 14,8
gef. : C 55,5 H 5,2 N 14,2

Das NMR-Spektrum zeigte ein Gewichts-Verhältnis von 78 : 22 der beiden Komponenten an.

NMR-Signale bei $\tau$ = 0,7 (1H), 2,2 (1H), 2,3-2,7 (8H), 2,9 (1H), 3,1 (1H), 4,45 (1H), 6,1 ppm (4H).

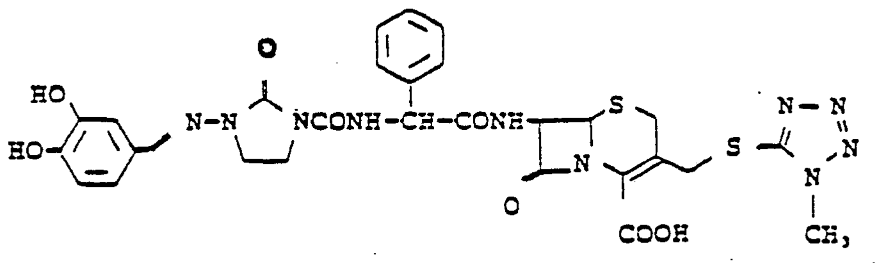

2 d.

10 mM (5,1 g, ~ 77 %ig) Säure von Versuch 2c und 3,6 (11 mM) 7-Amino-3'-(1-methyl-tetrazol-5-yl-thio)-cephalosporansäure wurden wie im Beispiel 1d in 47 % Ausbeute umgesetzt.

IR-Banden bei 3 600-2 400, 1 763, 1 713, 1 656, 1 518, 1 268, 1 218 $cm^{-1}$.

NMR-Signale des durch Gefriertrocknung der mit NaOH auf pH = 7,5 eingestellten Lösung der Säure gewonnenen Natriumsalzes bei $\tau$ = 2,2 (1H), 2,3-2,8 (6H), 2,9 (1H), 3,1 (1H), 4,15 (1H), 4,25 (1H), 5,03 (1H), 5,4 (1H), 5,7 (1H), 6,0 (3H), 6,1 (4H), 6,35 (1H), 6,5 ppm (1H).

OH Signale (breit) bei 2,1 und 3,5-7 ppm.

## Beispiel 3

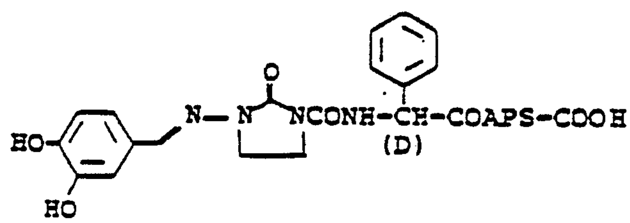

1,4 g (3,5 mM) Ampicillin-Trihydrat in 20 ml 80 %igem THF wurden bei pH 7 (mit 2 n NaOH konstant gehalten) mit 1 g (3,5 mM) Säurechlorid von Beispiel 2b bei Raumtemperatur umgesetzt. Nach pH-Konstanz wurde mit 30 ml Wasser versetzt, das THF abgezogen, 1 x mit Essigester gewaschen und dann mit 2 n HCl auf pH = 2 angesäuert. Die ausgefallene Penicillansäure (0,9 g) war im DC praktisch einheitlich.

IR-Banden bei 3 550-2 400, 1 768, 1 715, 1 635, 1 512, 1 269, 1 208 $cm^{-1}$.

NMR-Signale bei $\tau$ = 2,45-2,85 (7H), 3,1 (1H), 3,34 (1H), 4,5 (2H), 4,7 (1H), 5,8 (1H), 6,15- 6,5 (4H), 8,6 + 8,7 ppm (6H).

## Beispiel 4

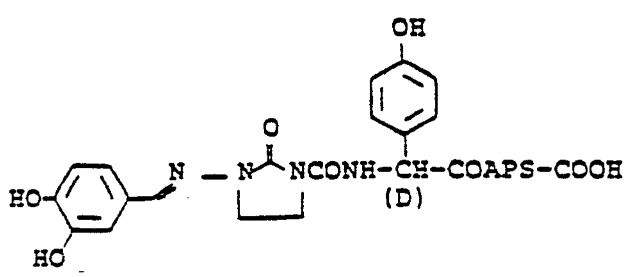

1,5 g (5 mM) Säurechlorid von Beispiel 2b, die 5 Min. mit Eiswasser gerührt und dann abgesaugt worden waren und 1,7 g Amoxicillin-Trihydrat wurden wie in Beispiel 3 beschrieben umgesetzt und als Penicillansäure isoliert. 2,0 g. Nach DC praktisch einheitlich.

IR-Banden bei 3 600-2 300, 1 770, 1 715, 1 665, 1 610, 1 512, 1 274, 1 216 $cm^{-1}$.

NMR-Signale bei τ = 2,4-3,4 (8H), 4,4-4,8 (3H), 5,7 (1H), 6,15 (4H), 8,35 + 8,45 ppm (6H).

Beispiel 5

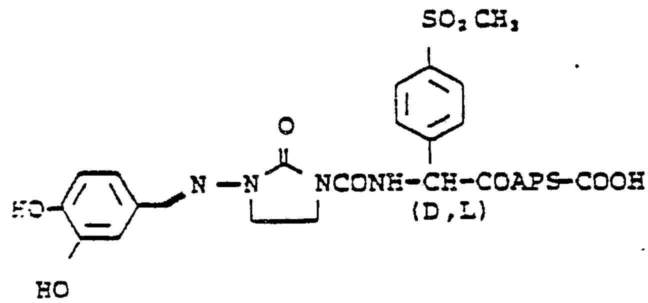

2,0 g (4,8 mM) Säurechlorid von Beispiel 2b und 4,6 mM D,L-α-Amino-4-mesyl-benzylpenicillin wurden wie in Beispiel 3 beschrieben zu 2,1 g Penicillansäure umgesetzt.

IR-Banden bei 3 600-2 200, 1 770, 1 718, 1 675, 1 517, 1 295, 1 275, 1 215, 1 147, 1 085, 959, 770 $cm^{-1}$.

NMR-Signale bei τ = 2,0 (4H), 2,2 (1H), 2,6 (1H), 2,9 (1H), 3,15 (1H), 3,95 (1H), 4,4 + 4,5 (2H), 5,75 (1H), 6,1 (4H), 6,8 (3H), 8,5 ppm (6H).

Beispiel 6

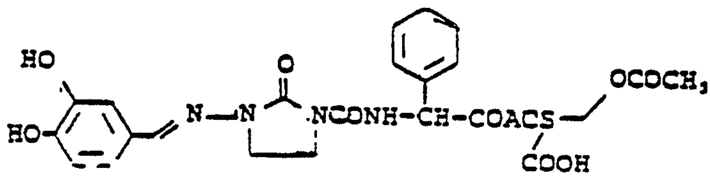

1,3 g (4,6 mM) Säurechlorid von Beispiel 2b und 1,86 g (4,6 mM) Cefaloglycin wurden wie in Beispiel 3 beschrieben in die Cefalosporinsäure überführt. 2,3 g.

IR-Banden bei 3 600-2 300, 1 770, 1 717, 1 520, 1 270, 1 224 $cm^{-1}$.

NMR-Signale bei τ = 2,3 (1H), 2,4-2,7 (6H), 2,95 (1H), 3,2 (1H), 4,2 (1H), 4,4 (1H), 4,95 + 5,0 (2H), 5,23 (1H), 6,0-6,25 (4H), 6,45 (1H), 6,65 (1H), 8,0 ppm (3H).

Beispiel 7

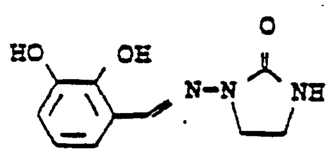

7 a.

Dieses Produkt wurde wie in Beispiel 1a beschrieben aus 2,3 -Dihydroxybenzaldehyd und Aminoimidazolidinon in praktisch quantitativer Ausbeute erhalten. Fp = 260 °C.

IR-Banden bei 3 400-2 500, 1 698, 1 260, 1 160, 730 $cm^{-1}$

ber.: C 54,3 H 5,0 N 19,0
gef.: C 54,2 H 5,0 N 18,6

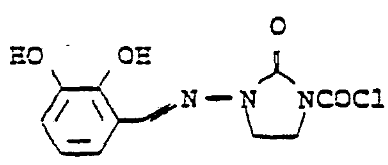

7b.

14,4 g Produkt von Beispiel 7a (75 mM) in 130 ml Essigester wurden unter Rühren bei 0 °C mit 6,05 ml (84 mM) Phosgen und dann innerhalb von 20 Min. tropfenweise mit 15,3 g (83 mM) Tributylamin versetzt. Nach dreistüdigem Rühren wurde der Niederschlag abgesaugt, mit Essigester gewaschen und abgepreßt. Man verrührte nun ca. 8 Min. mit 150 ml Wasser, saugte ab, wusch mit Wasser, saugte scharf ab und trocknete im Vak. Exsicc. über $P_2O_5$.

Ausbeute 14 g (66 %). Zers.-Punkt 210-212 °C.

Berechnet für 98 % Produkt + 2 % Tributylaminhydrochlorid

| | | | | |
|---|---|---|---|---|
| | C 47,0 | H 3,8 | Cl 12,6 | N 13,9 |
| gef. : | C 46,9 | H 3,3 | Cl 11,8 | N 14,1 |

IR-Banden bei 3 480, 1 862, 1 832, 1 800, 1 777, 1 708, 1 265, 1 236, 1 188, 1 163, 1 096, 984, 932, 823, 732 $cm^{-1}$.

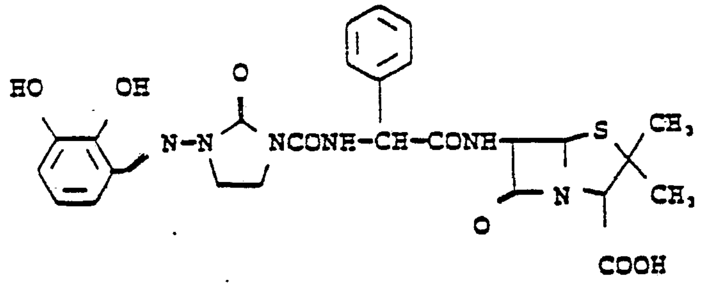

7 c.

1 g (3,5 mM) Säurechlorid des Beispiels 7b und 1,43 g (3,5 mM) Ampicillin-Trihydrat wurden wie bei Beispiel 8 umgesetzt.

Ausbeute 1,9 g. Das Produkt ist nach DC und NMR praktisch rein.

IR-Banden bei 3 600-2 400, 1 783, 1 730, 1 664, 1 527, 1 278, 1 240 und 736 $cm^{-1}$.

NMR-Signale bei $\tau$ = 0,7 (2H), 1,8 (1H), 2,37 (2H), 2,6 (3H), 2,9 (1H), 3,05 (1H), 3,2 (1H), 4,1 (1H), 4,26 (1H), 4,5 (1H), 5,7 (1H), 6,0 (4H), 8,4 (3H) und 8,5 ppm (3H).

## Beispiel 8

D-α-[2-Oxo-3-(3,4-dihydroxybenzylidenamino)-imidazolidin-1-yl-carbonylamino]-benzylpenicillin

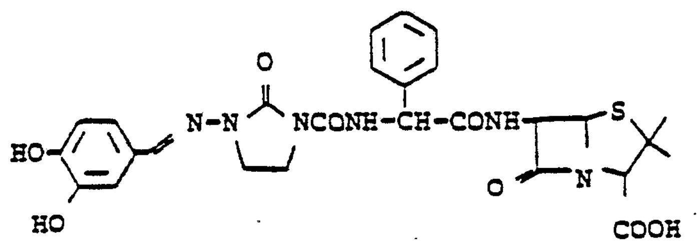

1,43 g (3,5 mM) Ampicillin-Trihydrat in 25 ml 80 %igem wäßrigem THF wurden mit 1 n NaOH auf pH = 7 gestellt und mit 1 g (2,9 mM) Säurechlorid von Beispiel 1b versetzt ; man rührte unter pH-Konstanz nach, bis keine NaOH bei pH 7 mehr verbraucht wurde. Nun wurde mit 20 ml Wasser versetzt, THF im Vak. abgezogen, 1 x mit Essigester extrahiert und die Penicillansäure mit 1 n HCl gefällt, abgesaugt, in frischem Wasser suspendiert und mit NaOH bei pH = 7 wieder in Lösung gebracht. Die filtrierte wäßrige Lösung wurde gefriergetrocknet.

Ausbeute : 1,5 g. DC praktisch einheitlich.

IR-Banden bei 3 600-2 250, 1 768, 1 710, 1 655, 1 517, 1 272, 1 210 $cm^{-1}$.

NMR-Signale bei $\tau$ = 0,8 (1H), 2,45 (1H), 2,55 (2H), 2,7 (4H), 3,05 (1H), 3,26 (1H), 4,47 (2H), 4,6 (1H), 5,7 (1H), 6,1-6,35 (4H), 8,5 (3H) und 8,57 ppm (3H).

Berechnet 98 % + 2 % $H_2O$ :

| | | | | |
|---|---|---|---|---|
| | C 51,4 | H 4,6 | N 13,3 | S 5,1 |
| gef. : | C 51,2 | H 5,2 | N 13,3 | S 5,0 |

Beispiel 9

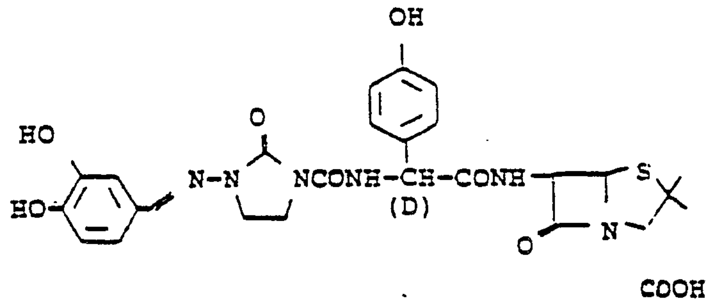

Wie im Beispiel 8 beschrieben wurden 6,4 mM Säurechlorid von Beispiel 1b mit 6,4 mMol Amoxicillin-Trihydrat zur Penicillansäure umgesetzt. Diese wurde mit 2 n NaOH in Wasser bei pH = 7 in Lösung gebracht, filtriert und gefriergetrocknet. 3,1 g (76 %). DC praktisch einheitlich.

IR-Banden bei 3 600-2 400, 1 770, 1 750, 1 715, 1 650, 1 595, 1 508, 1 270, 1 215 $cm^{-1}$.

NMR-Signale bei $\tau$ = 2,65 (4H), 3,1 (3H), 3,3 (1H), 4,55 (2H), 4,65 (1H), 5,8 (1H), 6,4 (4H), 8,5 (3H) und 8,58 ppm (3H).

Beispiel 10

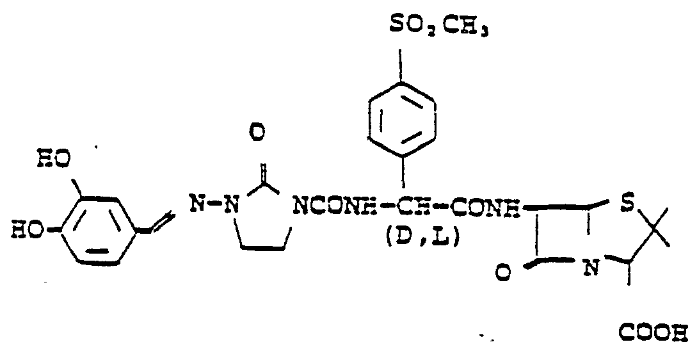

4,8 mM Säurechlorid von Beispiel 1b und D, L-p-Mesylampicillin wurden wie im Beispiel 8 umgesetzt, von einem neutralen Niederschlag (Dihydroxybenzylidenamino-imidazolidinon, 0,9 g) abgesaugt und als Säure gefällt und isoliert.

IR-Banden bei 3 600-2 400, 1 770, 1 717, 1 675, 1 617, 1 295, 1 272, 1 215 und 1 147 $cm^{-1}$.

NMR-Signale bei $\tau$ = 2,0 (4H), 2,2 (1H), 2,6 (1H), 2,9 (1H), 3,15 (1H), 3,95 (1H), 4,4 + 4,5 (2H), 5,75 (1H), 6,1 (4H), 6,8 (3H), 8,5 ppm (6H).

Die Aufspaltung der Signale bei $\tau$ = 5,75 und 8,5 zeigt ein D,L-Gemisch von ca. 1 : 1 an.

Beispiel 11

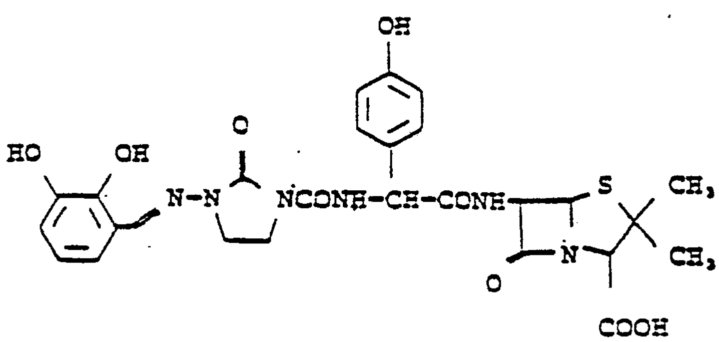

2,84 g (10 mM) Säurechlorid von Beispiel 7b und 4,2 g (10 mM) Amoxicillin-Trihydrat wurden wie Beispiel 8 umgesetzt.

Ausbeute 5 g.

IR-Banden bei 3 600-2 100, 1 770, 1 715, 1 648, 1 503, 1 270, 1 230, 1 205, 1 170, und 728 $cm^{-1}$.

NMR-Signale bei $\tau$ = 2,0 (1H), 2,6 (2H), 3,1 (5H), 4,4 (2H), 4,55 (1H), 5,8 (1H), 6,1 (4H) 8,37 (3H) und 8,47 ppm (3H).

Beispiel 12

7-D-α-[(2-Oxo-3-(3,4-dihydroxybenzylidenamino)-imidazolidin-1-yl)-carbonylamino]-phenylacetamido-cephalosporansäure

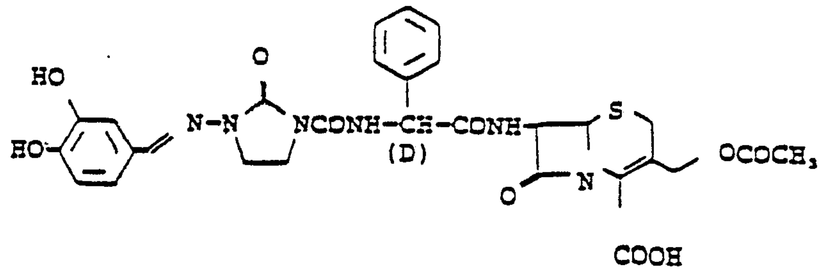

2 g (7 mM) Säurechlorid des Beispiels 2a wurden in der in Beispiel 8 beschriebenen Weise mit 2,8 g (7 mM) Cefaloglycin umgesetzt.
Ausbeute 2,8 g (55 %).
Nach DC praktisch einheitlich.

IR-Banden bei 3 550-2 250, 1 770, 1 710, 1 650, 1 517, 1 265, 1 215, 1 104 und 1 025 $cm^{-1}$.

NMR-Signale bei $\tau$ = 0,6 (2H), 2,16 (1H), 2,35 (2H), 2,55 (4H), 2,87 (1H), 3,07 (1H), 4,03 (1H), 4,14 (1H), 4,83 (1H), 4,9 (1H), 5,13 (1H), 6,03 (4H), 6,32 (1H), 6,48 (1H) und 7,9 ppm (3H).

Beispiel 13

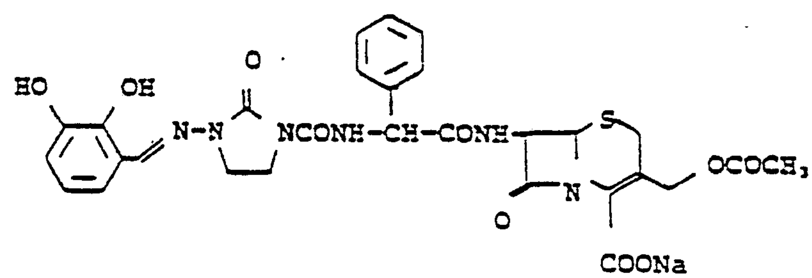

2,85 g (10 mM) Säurechlorid von Beispiel 7b und 4,05 g (10 mM) Cefaloglycin wurden wie im Beispiel 8 beschrieben in 26 % Ausbeute zur Cephalosporansäure umgesetzt, die nach DC praktisch einheitlich war und IR-Banden bei 3 600-2 250, 1 764, 1 718, 1 660, 1 515, 1 232, 1 027, 737 $cm^{-1}$ zeigte.

Nach Lösen mit NaOH bei pH = 7,5 in Wasser und Gefriertrocknen wurden 1,7 g des entsprechenden Natriumsalzes erhalten. IR-Banden bei 3 600-2 500, 1 770, 1 718, 1 659, 1 598, 1 520, 1 269, 1 230, 1 028 und 730 $cm^{-1}$.

NMR-Signale bei $\tau$ = 2,0 (1H), 2,4-2,65 (5H), 2,95-3,3 (3H), 4,2 (1H), 4,37 (1H), 5,0 (2H), 5,13 (1H), 6,1 (4H), 6,48 (1H), 6,75 (1H), 7,95 ppm (3H).

Beispiel 14

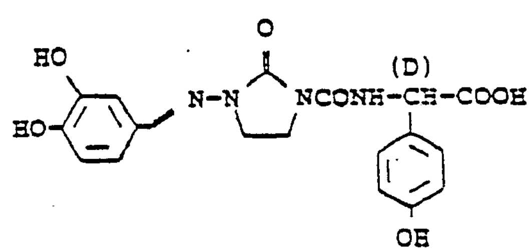

14 a.

Wie im Vorversuch wurden 5,4 g D-p-Hydroxyphenylglycin mit 7,4 g (26 mM) Säurechlorid des Beispiesl 2 a umgesetzt und nach Reaktionsende von 3 g ungelöstem Säurechlorid abfiltriert. Nach Ansäuern erhielt man 11,7 g Säure.

IR-Banden bei 3 550-2 000, 1 714, 1 642, 1 538, 1 508, 1 266, 1 210 und 1 170 $cm^{-1}$.

NMR-Signale bei = 0,9 (1H), 2,2 (1H), 2,64 (3H) 2,9 (1H), 3,1 (1H), 4,63 (1H) und 6,1 ppm (4H).

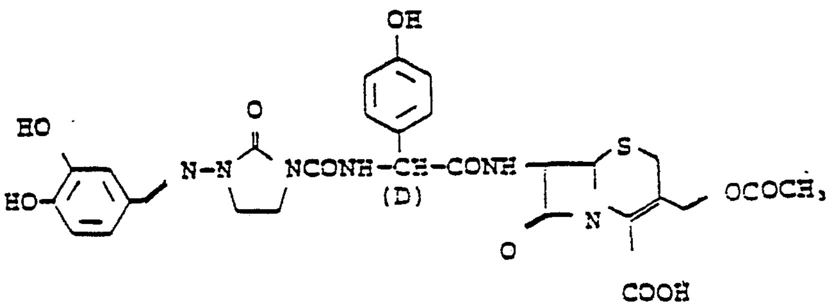

14 b.

1 g Säure von Beispiel 14a wurden mit 2,2 mN (0,6 g) 7-Aminocephalosporansäure wie in Beispiel 1d beschrieben umgesetzt. Ausbeute : 0,9 g.

IR-Banden bei 3 550-2 150, 1 763, 1 710, 1 645, 1 602, 1 508, 1 260, 1 210, 1 168, 1 103, 1 025, 739 und 719 $cm^{-1}$.

NMR-Signale bei $\tau$ = 0,8 (2H), 2,2 (1H), 2,6 (3H), 2,9 (1H), 3,1 (3H), 4,2 (1H), 4,3 (1H), 4,9-5,03 (3H), 6,1 (4H), 6,5 (1H), 6,7 (1H), 7,95 (1H), OH-Signale (breit) bei 2,1, 2,6, 5,7 und 6,4 ppm.

## Beispiel 15

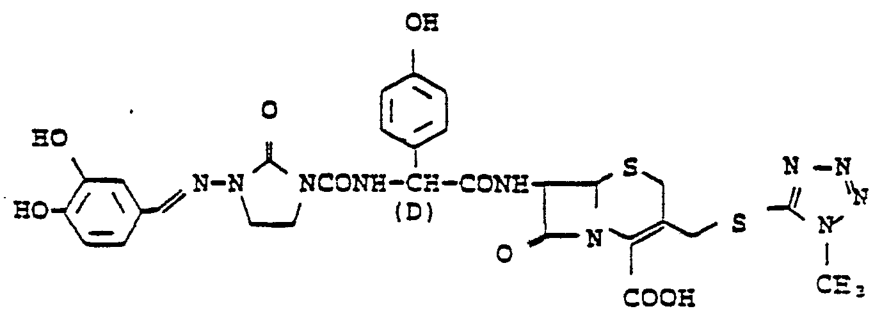

10 mM Säure von Beispiel 14a und 11 mM 7-Amino-3'-(1-methyl-tetrazol-5-yl-thio)-cephalosporansäure wurden wie in Beispiel 1d beschrieben in 80 % Ausbeute umgesetzt.

IR-Banden bei 3 600-2 250, 1 770, 1 716, 1 652, 1 608, 1 510, 1 265, 1 210, 1 170, 1 100 $cm^{-1}$.

NMR-Signale bei $\tau$ = 0,8 (2H), 2,15 (1H), 2,6 (3H), 2,9 (1H), 3,1 (3H), 4,1 (1H), 4,3 (1H), 4,85 (1H), 5,55 (2H), 5,9 (3H), 6,05 (4H) und 6,2 ppm (2H).

## Beispiel 16

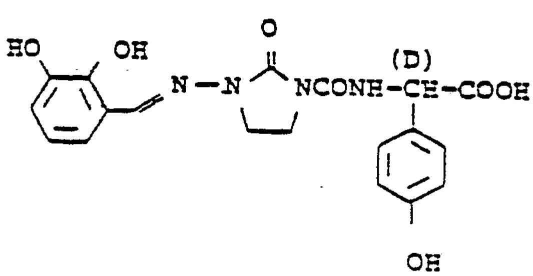

16 a.

Wie in Beispiel 2c wurden 4,5 g (16 mM) Säurechlorid von Beispiel 7b und 2,7 g (16 mM) D-p-Hydroxyphenylglycin umgesetzt und von 0,5 g unumgesetztem Säurechlorid abgesaugt. Nach Ansäuern erhielt man 4 g Produkt.

IR-Banden bei 3 500-2 100, 1 718, 1 650, 1 538, 1 267, 1 168 und 734 $cm^{-1}$.

NMR-Signale bei $\tau$ = 0,95 (1H), 1,8 (1H), 2,67 (2H), 2,95 (1H), 3,05 (1H), 3,1 (2H), 3,2 (1H), 4,6 (1H) und 6,0 ppm (4H).

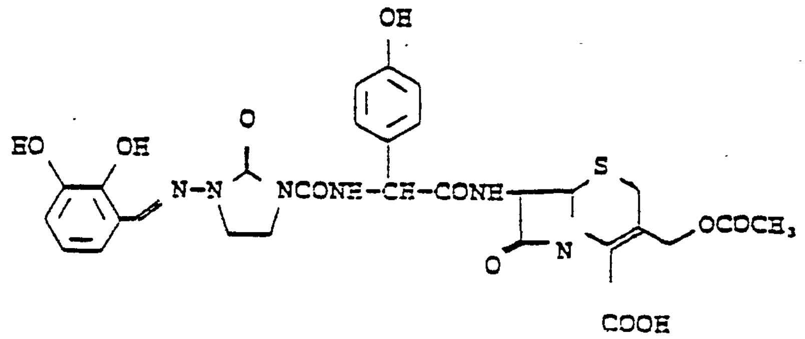

16 b.

2,1 g (5 mM) Carbonsäure aus Beispiel 16a und 1,5 g 7-Aminocephalosporansäure wurden wie in Beispiel 1d beschrieben zu 2,9 g Cephalosporansäure umgesetzt, die anschließend in das gefriergetrocknete Na-Salz überführt wurde. 2,9 g.

IR-Banden bei 3 600-2 400, 1 770 (Schulter), 1 717, 1 659, 1 559, 1 507, 1 270, 1 235, 1 028, 735 $cm^{-1}$.

NMR-Signale bei $\tau$ = 0,76 (1H), 0,85 (1H), 1,82 (1H), 2,6 (2H), 2,9 (1H), 3,05 (1H), 3,13 (2H), 3,24 (1H), 4,3 (2H), 4,9 (1H), 5,04 (2H), 6,04 (4H), 6,54 (1H), 6,74 (1H) und 7,97 ppm (3H).

Beispiel 17

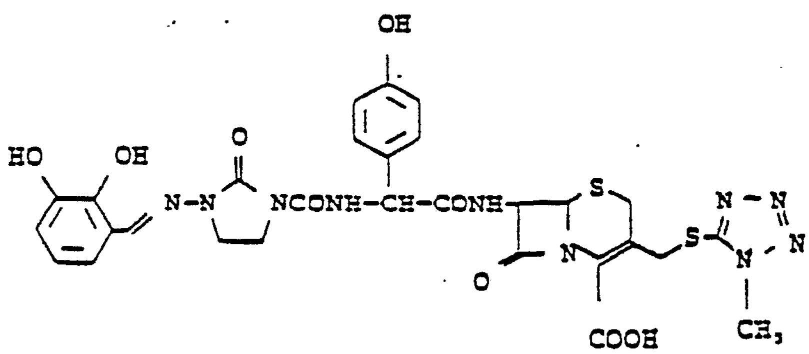

1,7 g (4 mM) Carbonsäure aus Beispiel 16a und 1,45 g (4,4 mM) 7-Amino-3'-(1-methyltetrazol-5-yl-thio)-cephalosporansäure wurden wie in Beispiel 1d beschrieben zu 2,1 g Cephalosporansäure umgesetzt, die nochmals bei pH = 7,5 mit NaOH in Wasser gelöst und erneut mit HCl ausgefällt wurde. Anschließend wurde in das gefriergetrocknete Natriumsalz (1,7 g) überführt.

IR-Banden bei 3 600-2 500, 1 770 (Schulter), 1 722 (st), 1 648, 1 598, 1 518, 1 272, 1 238, 1 168 und 735 $cm^{-1}$.

NMR-Signale bei $\tau$ = 1,97 (1H), 2,6 (2H), 2,97 (1H), 3,08 (1H), 3,14 (2H), 3,2 (1H), 4,27 (1H), 4,5 (1H), 5,0 (1H), 5,6 (1H), 5,7 (1H), 5,96 (3H), 6,05 (4H), 6,33 (1H) und 6,6 ppm (1H).

Beispiel 18

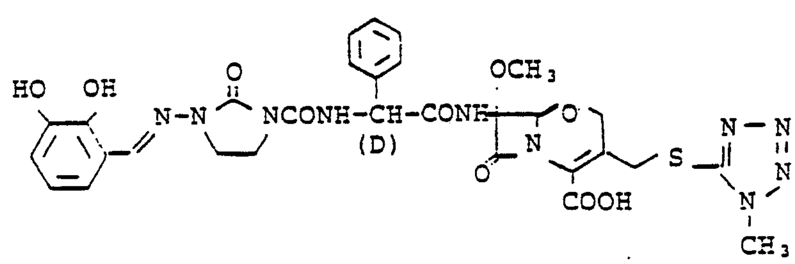

285 mg (1 mM) Säurechlorid von Beispiel 7b und 460 mg (1 mM) 7β-(D-α-Amino-phenylacetamido)-

7α-methoxy-3-(1-methyltetrazol-5-yl-thiomethyl)-1-dethia-1-oxa-cephemcarbonsäure wurden in der in Beispiel 8 beschriebenen Weise in 32 % Ausbeute zum 1-Dethia-1-oxa-cephalosporin umgesetzt.

IR-Banden bei 3 600-2 250, 1 770, 1 720, 1 660, 1 510 und 740 $cm^{-1}$.

NMR-Signale bei τ = 2,1 (1H), 2,4-2,7 (5H), 2,9-3,3 (3H), 4,4 (1H), 4,9 (1H), 5,4-5,8 (4H), 6,0 (3H), 6,1 (4H), 6,6 ppm (3H) ($DMF-d_7$-$DMSO-d_6$).

Beispiel 19

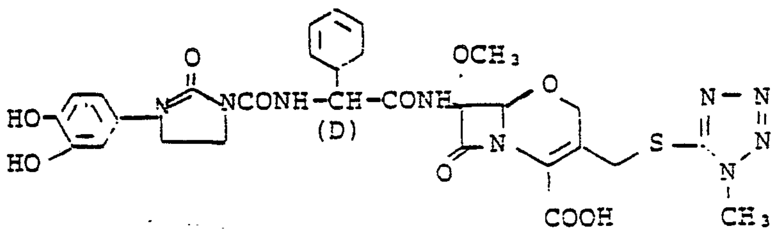

341 mg (1 mM) Säurechlorid von Beispiel 1 b und 460 mg (1 mM) 7β-(D-α-Amino-phenylacetamido)-7α-methoxy-3-(1-methyltetrazol-5-yl-thiomethyl)-1-dethia-1-oxa-cephemcarbonsäure wurden in der im Beispiel 8 beschriebenen Weise in 48 % Ausbeute zum 1-Dethia-1-oxa-cephalosporin umgesetzt.

IR-Banden bei 3 500-2 200, 1 771, 1 715, 1 662, 1 515 und 728 $cm^{-1}$.

NMR-Signale bei τ = 2,2 (1H), 2,3-2,8 (6H), 2,9 (1H), 3,1 (1H), 4,2 (1H), 4,85 (1H), 5,3-5,7 (4H), 6,0 (3H), 6,1 (4H), 6,6 ppm (3H).

Beispiel 20

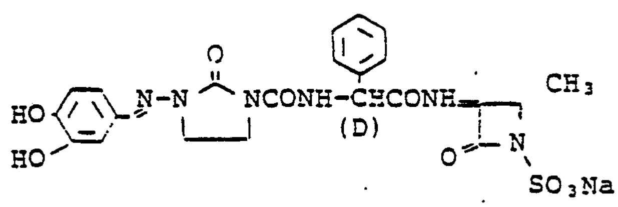

0,8 g (2 mM) Säure von Beispiel 2c wurden wie im Beispiel 1d beschrieben unter Verwendung von Aceton anstatt DMF und von Triethylamin und etwas 3-Dimethylaminopropanol anstatt N-Methylmorpholin mit 400 mg (2,2 mM) 3-β-Amino-4-α-methyl-azetidin-2-on-1-sulfonsäure umgesetzt. Die Reaktionslösung wurde mit Wasser versetzt, im Vakuum vom Lösungsmittel befreit, auf pH = 3 gestellt und von einem Niederschlag abgesaugt. Die wäßrige Phase wurde gefriergetrocknet. 0,9 g.

Dieses Produkt wurde 20 Min. mit 10 ml $CH_2Cl_2$ ausgerührt, abgesaugt und im Exsiccator getrocknet.

IR-Banden bei 3 550-2 500, 1 770, 1 755, 1 715, 1 665, 1 620 $cm^{-1}$.

NMR-Signale bei τ = 2,3 (1H), 2,45-2,7 (6H), 2,95 (1H), 3,2 (1H), 4,6 (1H), 5,4 (1H), 5,95 (1H), 6,1 (4H) und 8,4 ppm (3H).

Beispiel 21

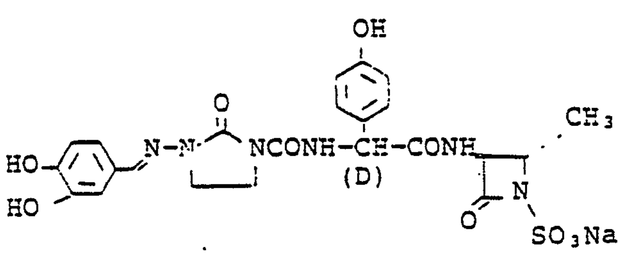

1 g (2,5 mM) Säure von Beispiel 14a wurde wie in Beispiel 20 angegeben mit 490 mg (2,7 mM) 3-β-Amino-4-α-methyl-azetidin-2-on-1-sulfosäure umgesetzt.

Ausbeute 450 mg.

IR-Banden bei 3 600-2 400, 1 755, 1 660, 1 510 und 1 042 $cm^{-1}$.

NMR-Signale bei $\tau$ = 2,3 (1H), 2,7 (3H), 2,9 (1H), 3,2 (3H), 4,5 (1H), 5,25 (1H), 5,9 (1H), 6,1 (4H), 8,4 ppm (3H).

Beispiel 22

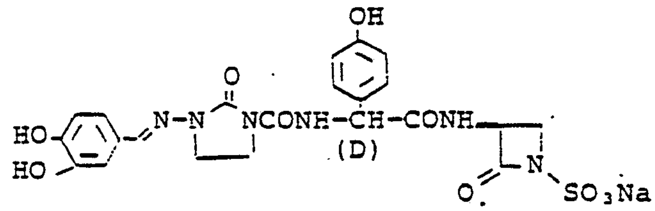

1 g (2,5 mM) Säure von Beispiel 14a wurde wie in Beispiel 20 angegeben mit 2,7 mM (450 mg) 3-β-Amino-azetidin-2-on-1-sulfosäure umgesetzt.
Ausbeute : 450 mg.

IR-Banden bei 3 600-2 300, 1 770, 1 755, 1 657, 1 042 $cm^{-1}$.

NMR-Signale bei $\tau$ = 2,3 (1H), 2,6-2,7 (3H), 2,9 (1H), 3,1-3,2 (3H), 4,5 (1H), 5,1 (1H), 6,0-6,2 (5H) und 6,4 ppm (1H).

Beispiel 23

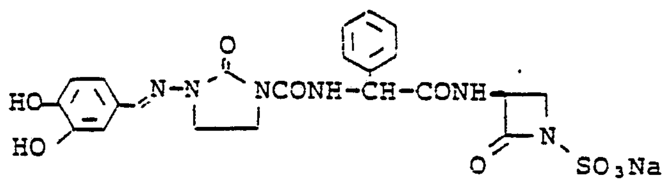

0,8 g (2 mM) Säure von Beispiel 2c wurden wie in Beispiel 20 angegeben mit 365 mg (2,2 mM) 3-β-Amino-azetidin-2-on-1-sulfosäure umgesetzt. Ausbeute : 0,6 g.

IR-Banden bei 3 600-2 300, 1 770, 1 757, 1 717, 1 660, 1 520 und 1 044 $cm^{-1}$.

NMR-Signale bei $\tau$ = 2,3 (1H), 2,45-2,7 (6H), 2,95 (1H), 3,2 (1H), 4,5 (1H), 5,1 (1H), 6,0-6,2 (5H) und 6,4 ppm (1H).

Beispiel 24

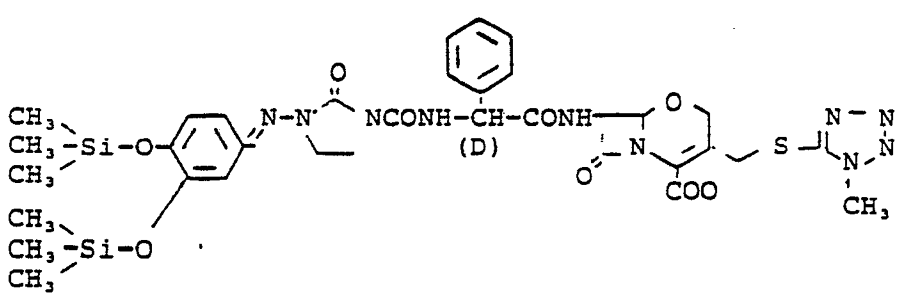

479 mg (0.88 mM, 1.3 äqu.) disilylierte Präcusorsäure von Beispiel 2 c in 2.8 ml trockenem Methylenchlorid wurde unter Stickstoff auf 0 °C gekühlt, mit 116 mcl (1.06 mM, 1.56 äqu.) N-Methylmorpholin versetzt und nach 3 Min. Rühren auf — 35 °C gekühlt. Nun versetzte man mit 163 mcl (0.97 mM, 1.43 äqu.) Trifluormethansulfonsäureanhydrid und rührte 20 Min. bei dieser Temperatur. Lsg. A.

Inzwischen löste man 325 mg (0.68 mM) 7-Amino-1-dethia-1-oxa-3'-desacetoxi-3'-(1-methyl-tetrazol-5-yl)-thio-cephalosporan-säurebenzhydrylester unter Zusatz von 0.75 mM (1.1 äqu.) N-Methylmorpholin in 2.2 ml $CH_2Cl_2$ und drückte diese Lösung zur — 55 °C kalten Lsg. A.

Man ließ die Mischung auf —20 °C erwärmen, goß auf wäßrige $NaHCO_3$— Lsg., die mit $CHCl_3$ überschichtet war, wobei heftig gerührt wurde. Der organische Extrakt wurde kurz mit Wasser gewaschen, über $MgSO_4$ getrocknet und eingedampft.

Ausbeute : 168 mg (25 %).

NMR-Signale bei δ = 9,1 (1H), 7,54 (1H), 7,5 (1H), 7,45-7,15 (17H), 6,85 (1H), 6,8 (2H), 5,7 (1H), 5,5 (1H), 5,0 (1H), 4,65 (1H), 4,45 (1H), 4,24 (2H), 4,0 (2H), 3,77 (5H) und 0,3 ppm (18H) (in $CDCl_3$).

IR-Banden bei 3 300, 3 065, 3 035, 2 961, 1 796, 1 730, 1 678, 1 510, 1 410, 1 254, 910, 844, 746, 698 $cm^{-1}$ (in KBr).

Beispiel 25

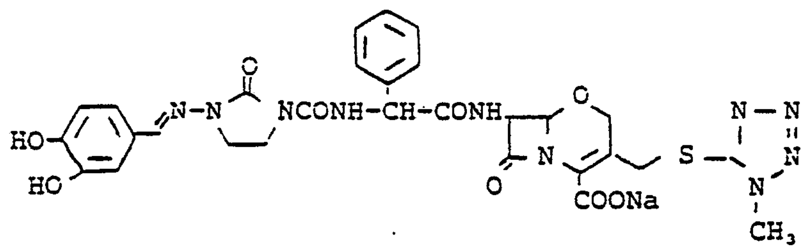

151 mg des Produktes von Beispiel 24 wurden in 6 ml $CH_2Cl_2$ bei 0 °C mit 3 ml Anisol und 3 ml Trifluoressigsaure 30 Min. gerührt. Es wurde mit Wasser versetzt, die $CH_2Cl_2$-Phase abgetrennt, die wäßrige Phase mit einigen Millilitern $CH_2Cl_2$ extrahiert und die vereinigten organischen Phasen mit Wasser gewaschen. Man trocknete über $MgSO_4$, zog im Vakuum Trifluoressigsäure und Methylenchlorid ab, versetzt wieder mit $CH_2Cl_2$ und extrahierte das Produkt in Wasser indem man mit 1 n NaOH pH = 7 einstellte. Die wäßrige Phase wurde gefriergetrocknet.

Ausbeute 98 mg = 91 % d. Th.

IR-Banden bei 1 771, 1 725, 1 668, 1 604, 1 528, 1 459, 1 417, 1 274, 1 103 und 1 023 $cm^{-1}$ (in KBr).

NMR-Signale bei δ = 9,14 (1H), 9,05 (1H), 7,6 (1H), 7,45-7,3 (5H), 7,16 (1H), 6,9 (1H), 6,73 (1H), 5,62 (1H), 5,35 (1H), 4,9 (1H), 4,45 (1H), 4,32 (2H), 4,1 (1H), 3,9 (3H) und 3,76 ppm (4H).

**Patentansprüche**

1. Verbindungen der Formel I,

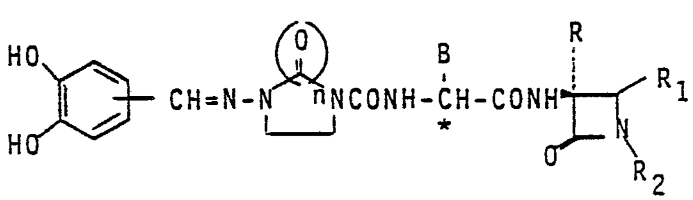

(I)

in welcher

B für gegebenenfalls durch Methyl, Ethyl, Aminomethyl, Hydroxy, Methoxy, Ethoxy, Carbamoyloxy, Acetoxy, Amino, Mesylamino, Methylamino, Aminosulfonylamino, Guanidyl, Carbamoylamino, Carboxy, Methoxycarbonyl, Carbamoyl, Amidino, Mesyl, Methylsulfinyl, Sulfo, Methylthio oder Halogen einfach oder doppelt substituiertes Phenyl oder Cyclohexadienyl oder für einen ungesättigten gegebenenfalls durch Methyl, Ethyl, Hydroxy, Oxo, Amino, Imino, Mesyl, Mesylamino, Carboxy, Carbamoyl, Acetyl mono- oder disubstituierten 5- oder 6-gliedrigen Heterocyclus mit 1-4 Heteroatomen, die Sauerstoff, Stickstoff oder Schwefel sein können, steht ;

R für Wasserstoff oder Methoxy steht ;

n 1 oder 2 ist ;

$R_1$ Wasserstoff oder gegebenenfalls durch Hydroxy, Amino, Carboxy, Carbamoyl oder Mesyl substituiertes Alkyl mit bis zu 5 C-Atomen, das ungesättigt oder cyclisiert sein kann, ist ;

$R_2$ $SO_3^{\ominus}M^{\oplus}$ ist ;

$M^{\oplus}$ ein Proton oder ein Kation ist ;

oder $R_1$ und $R_2$ zusammen mit dem Azetidinonring, an den sie gebunden sind, für

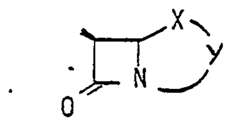

stehen, worin

X für S, O, SO, $SO_2$ oder $CH_2$ steht ; und

Y für die Gruppen

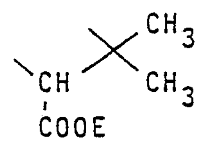

und

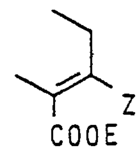

steht,

in welchen das Kohlenstoffatom, das die Gruppe —COOE trägt, an das Stickstoffatom des β-Lactamrings gebunden ist und

Z für Wasserstoff, Halogen, $C_{1-3}$-Alkoxy oder $—CH_2—T$ steht,

T Wasserstoff, Acetat, Propionat, n- und i-Butyrat, die durch Carboxy, Hydroxy oder Amino substituiert sein können, Pyridinium, Carboxamidopyridinium, Aminopyridinium, Carbamoyloxy, Azido, Cyano, Hydroxy, die Gruppe —S-Phenyl, welche durch Methyl, Halogen, Amino, Hydroxy oder Carboxy substituiert sein kann, oder die Gruppe —S-Het bedeutet, in welcher Het für einen gegebenenfalls durch Alkyl mit bis zu 3 C-Atomen, welches durch Carboxy, Sulfo, Amino, Methylamino, Dimethylamino oder Hydroxy substituiert sein kann, durch Hydroxy, Oxo, Amino, Imino oder Sulfo einfach oder zweifach substituierten heterocyclischen 5- oder 6-gliedrigen Ring mit 1-4 Heteroatomen, die Sauerstoff, Stickstoff oder Schwefel sein können, steht ;

und wobei

E für Wasserstoff, eine pharmazeutisch verwendbare Estergruppierung, ein salzbildendes Kation oder eine geeignete Schutzgruppe steht ;

wobei diese Verbindungen der Formel I bezüglich des Chiralitätszentrums $\overset{*}{C}$ in den beiden möglichen R- und S-Konfigurationen sowie als Gemische der daraus resultierenden Diastereomeren vorliegen können, und wobei die Verbindungen der Formel I bezüglich der Iminogruppe sowohl in der syn-Form als auch in der anti-Form vorliegen können und wobei diese Verbindungen der Formel I auch in den verschiedenen Hydratformen vorliegen können.

2. Verbindungen gemäß Anspruch 1 der Formel I, in welcher

n 1 bedeutet und

$R_2$ $SO_3—M^+$ bedeutet, worin $M^+$ ein Alkali- oder Erdalkalikation, das Aluminiumkation oder das Ammoniumion, ein protoniertes primäres, sekundäres und tertiäres aliphatisches Amin oder ein heterocyclisches Amin bedeutet.

3. Verbindungen nach Anspruch 1, in denen

B Phenyl, welches unsubstituiert oder durch Methyl, Ethyl, Aminomethyl, Hydroxy, Methoxy, Ethoxy, Carbamoyloxy, Acetoxy, Amino, Mesylamino, Methylamino, Aminosulfonylamino, Guanidyl, Carbamoylamino, Carboxy, Methoxycarbonyl, Carbamoyl, Amidino, Mesyl, Methylsulfinyl, Sulfo, Methylthio, oder Halogen einfach oder doppelt substituiert ist, oder ein Furyl-, Methylfuryl-, Thienyl, Methylthienyl-, 2-Aminothiazolyl, Thiazolyl-, Methylisoxazolyl-, Isoxazolyl, Pyridyl-, 2-Aminopyrimidyl-, Thiadiazolyl-, Pyranyl-, Thiapyranyl- oder Sydnonylring ist, und

T Wasserstoff, Acetat, Propionat, n- und i-Butyrat, die durch Carboxy, Hydroxy oder Amino substituiert sein können, Pyridinium, Carboxamidopyridinium, Aminopyridinium, Carbamoyloxy, Azido, Cyano, Hydroxy, die Gruppe —S-Phenyl, welche durch Methyl, Halogen, Amino, Hydroxy, Carboxy substituiert sein kann, oder die Gruppe —S-Het bedeutet, in welcher Het den Thiazol-, Isothiazol-, Thiadiazolyl-, Thiazolyl- oder Tetrazolylring, jeweils unsubstituiert oder durch Methyl, Sulfomethyl, Carboxymethyl, Dimethylaminoethyl substituiert, den Pyridyl-, 1-Oxido-pyridyl-, 2-Methyl-5-oxo-6-hydroxy-1,4-dihydro-1,2,4-Triazin- oder den 4-Formylmethyl-5-oxo-6-hydroxy-4,5-dihydro-1,2,4-triazinylring bedeutet.

4. Verfahren zur Herstellung von Verbindungen gemäß Anspruch 1, dadurch gekennzeichnet, daß man 3-(Aminoacetamido)-azetidin-2-one der Formel III

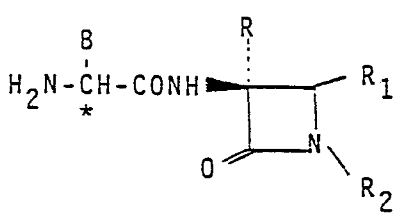

(III)

in der B, R, $R_1$, $R_2$, $M^{\oplus}$, X, Y, E, Z und T die in Anspruch 1 gegebenen Bedeutungen haben, wobei diese Verbindungen der Formel III bezüglich des Chiralitätszentrums $\overset{*}{C}$ in den beiden möglichen R- und S-Konfigurationen sowie als Gemische der daraus resultierenden Diastereomeren vorliegen können, mit Verbindungen der allgemeinen Formel II

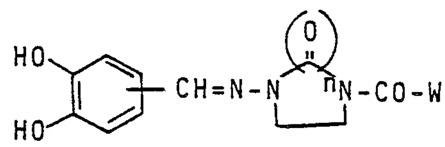

(II)

in welcher

W für Halogen, Azid oder eine andere nukleofuge Abgangsgruppe steht,

n 1 oder 2 ist und in welcher die beiden Hydroxygruppen durch Silyl- oder Cyclosilylgruppen geschützt sein können,

in Gegenwart eines Lösungsmittels und gegebenenfalls eines Säurebindemittels bei Temperaturen von etwa —20 °C bis etwa + 50 °C umsetzt und die erhaltenen β-Lactam-Antibiotika gegebenenfalls von den vorhandenen Schutzgruppen befreit und in ihre pharmazeutisch verwendbaren Salze oder Ester überführt oder aus den erhaltenen Salzen gewünschtenfalls die freien Säuren herstellt.

5. Verfahren zur Herstellung von Verbindungen gemäß Anspruch 1, dadurch gekennzeichnet, daß man 3-Aminoazetidin-2-one der Formel IV

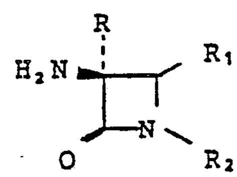

(IV)

in welcher R, $R_1$ und $R_2$ die in Anspruch 1 angegebene Bedeutung haben, mit einem acylierend wirkenden Derivat einer vic-Dihydroxyphenylmethylenamino-(2-oxo-imidazolidin)- oder -(2,3-dioxo-piperazin)-1-carbonylaminoessigsäure der Formel V

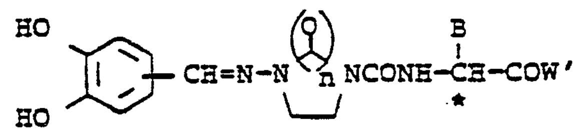

(V)

in welcher n, B und $\overset{*}{C}$ die oben angegebene Bedeutung haben und W' OH oder eine reaktive Abgangsgruppe ist umsetzt und gegebenenfalls nach Abspaltung vorhandener Schutzgruppen pharmazeutisch verwendbare Salze oder Ester herstellt oder aus den erhaltenen Salzen gewünschtenfalls die freien Säuren herstellt.

6. Verfahren zur Herstellung von Verbindungen gemäß Anspruch 1 der allgemeinen Formel VI,

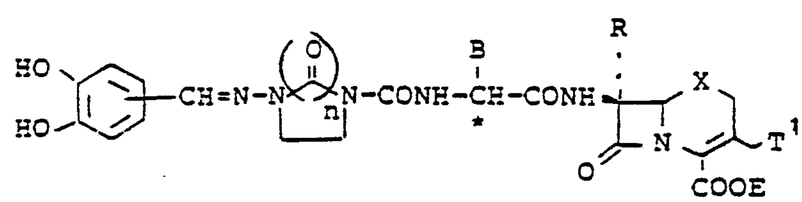

(VI)

in der n, B, $\overset{*}{C}$, R, X und E die in Anspruch 1 angegebene Bedeutung haben und T' die Bedeutung von T in Anspruch 1 außer Wasserstoff hat, dadurch gekennzeichnet, daß man eine Verbindung der allgemeinen Formel VII

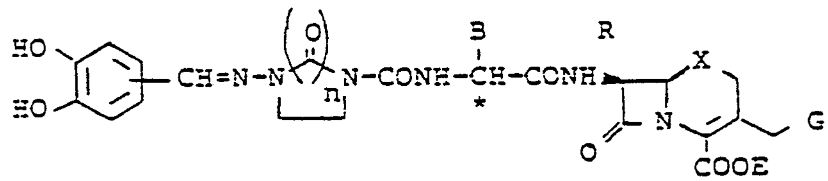

(VII)

in der n, B, C*, R, X und E die in Anspruch 1 angegebene Bedeutung haben und G eine nukleofuge Gruppe wie Halogen, Pseudohalogen, Acetoxy, Dichloracetoxy, Mesyloxy bedeutet, mit einem Nukleophil [T'], in dem T' die oben angegebene Bedeutung hat, unter Austritt von [G] zur Umsetzung bringt und gegebenenfalls nach Abspaltung vorhandener Schutzgruppen in die pharmazeutisch verwendbaren Salze oder Ester überführt oder aus den erhaltenen Salzen gewünschtenfalls die freien Säuren herstellt.

7. Verwendung der Verbindungen der Ansprüche 1-3 zur Herstellung von Arzneimitteln.

8. Verbindungen nach den Ansprüchen 1-3 zur Verwendung in der Wachstumsförderung von Tieren.

9. Tierfutterzusätze, enthaltend Verbindungen nach den Ansprüchen 1-3.

10. Arzneimittel, enthaltend Verbindungen nach den Ansprüchen 1-3.

**Claims**

1. Compounds of the formula I

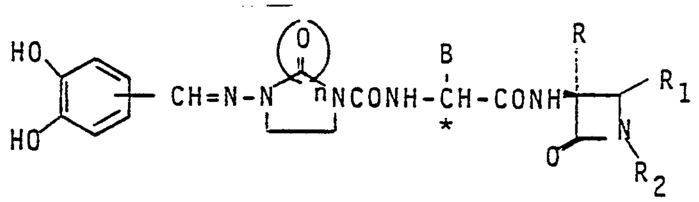

(I)

in which

B represents cyclohexadienyl or phenyl which is optionally mono- or disubstituted by methyl, ethyl, aminomethyl, hydroxyl, methoxy, ethoxy, carbamoyloxy, acetoxy, amino, mesylamino, methylamino, aminosulphonylamino, guanidyl, carbamoylamino, carboxyl, methoxycarbonyl, carbamoyl, amidino, mesyl, methylsulphinyl, sulpho, methylthio or halogen, or an unsaturated 5- oder 6-membered heterocyclic structure which has 1-4 hetero atoms, which can be oxygen, nitrogen or sulphur, and is optionally mono- or disubstituted by methyl, ethyl, hydroxyl, oxo, amino, imino, mesyl, mesylamino, carboxyl, carbamoyl or acetyl ;

R represents hydrogen or methoxy ;

n is 1 or 2 ;

$R_1$ is hydrogen or alkyl which has up to 5 C atoms, is optionally substituted by hydroxyl, amino, carboxyl, carbamoyl or mesyl and can be unsaturated or cyclised ;

$R_2$ is $SO_3^{\ominus}M^{\oplus}$ ;

$M^{\oplus}$ is a proton or a cation

or $R_1$ and $R_2$, together with the azetidinone ring to which they are bonded, represent

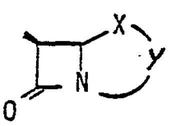

wherein

X represents S, O, SO, $SO_2$ or $CH_2$ ; and

Y represents the groups

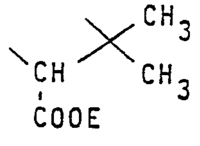

and

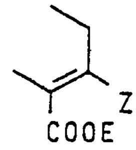

in which the carbon atom which carries the group —COOE is bonded to the nitrogen atom of the β-lactam ring and

Z represents hydrogen, halogen, $C_{1-3}$-alkoxy or $—CH_2—T$,

T denotes hydrogen, acetate, propionate, n- and i-butyrate, which can be substituted by carboxyl, hydroxyl or amino, pyridinium, carboxamidopyridinium, aminopyridinium, carbamoyloxy, azido, cyano, hydroxyl, the group —S-phenyl which can be substituted by methyl, halogen, amino, hydroxyl or carboxyl, or the group —S-Het, in which Het represents a heterocyclic 5- or 6-membered ring which has 1-4 hetero atoms, which can be oxygen, nitrogen or sulphur, and is optionally mono- or disubstituted by alkyl with up to 3 C atoms which can be substituted by carboxyl, sulpho, amino, methylamino, dimethylamino or hydroxyl, or by hydroxyl, oxo, amino, imino or sulpho ;

and wherein

E represents hydrogen, a pharmaceutically usable ester grouping, a salt-forming cation or a suitable protective group ;
it being possible for these compounds of the formula I, with respect to the chirality centre $\overset{*}{C}$, to be present in the two possible R and S configurations and in the form of mixtures of the diastereomers resulting therefrom, and it being possible for the compounds of the formula I, with respect to the imino group, to be present both in the syn form and in the anti form, and it being possible for these compounds of the formula I also to be present in the various hydrate forms.

2. Compounds according to Claim 1 of the formula I, in which
n denotes 1 and
$R_2$ denotes $SO_3^-M^+$, wherein $M^+$ denotes an alkali metal cation or alkaline earth metal cation, the aluminium cation or the ammonium ion, a protonated primary, secondary and tertiary aliphatic amine or a heterocyclic amine.

3. Compounds according to Claim 1, in which
B is phenyl which is unsubstituted or mono- or disubstituted by methyl, ethyl, aminomethyl, hydroxyl, methoxy, ethoxy, carbamoyloxy, acetoxy, amino, mesylamino, methylamino, aminosulphonylamino, guanidyl, carbamoylamino, carboxyl, methoxycarbonyl, carbamoyl, amidino, mesyl, methylsulphinyl, sulpho, methylthio or halogen, or a furyl, methylfuryl, thienyl, methylthienyl, 2-aminothiazolyl, thiazolyl, methylisoxazolyl, isoxazolyl, pyridyl, 2-aminopyrimidyl, thiadiazolyl, pyranyl, thiapyranyl or sydnonyl ring, and
T denotes hydrogen, acetate, propionate, n- and i-butyrate, which can be substituted by carboxyl, hydroxyl or amino, pyridinium, carboxamidopyridinium, aminopyridinium, carbamoyloxy, azido, cyano, hydroxyl, the group —S-phenyl which can be substituted by methyl, halogen, amino, hydroxyl or carboxyl, or the group —S-Het, in which Het denotes the thiazole, isothiazole, thiadiazolyl, thiazolyl or tetrazolyl ring, in each case unsubstituted or substituted by methyl, sulphomethyl, carboxymethyl or dimethylaminoethyl, the pyridyl, 1-oxido-pyridyl, 2-methyl-5-oxo-6-hydroxy-1,4-dihydro-1,2,4-triazine or the 4-formylmethyl-5-oxo-6-hydroxy-4,5-dihydro-1,2,4-triazinyl ring.

4. Process for the preparation of compounds according to Claim 1, characterised in that 3-(amino-acetamido)-azetidin-2-ones of the formula III

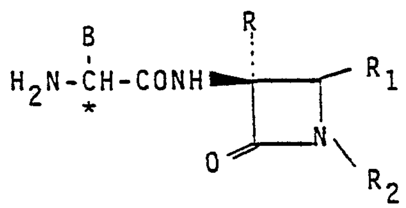

(III)

in which B, R, $R_1$, $R_2$, $M^{\oplus}$, X, Y, E, Z and T have the meanings given in Claim 1, it being possible for these compounds of the formula III, with respect to the chirality centre $\overset{*}{C}$, to be present in the two possible R and S configurations and in the form of mixtures of the diastereomers resulting therefrom, are reacted with compounds of the general formula II

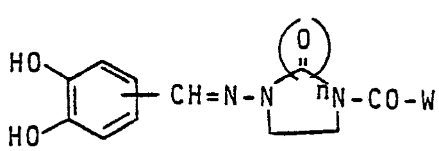

(II)

in which
W represents hydrogen, azide or another nucleofugic leaving group, and
n is 1 or 2 and in which the two hydroxyl groups can be protected by silyl or cyclosilyl groups, in the presence of a solvent and, if appropriate, an acid-binding agent, at temperatures of about — 20 °C to about + 50 °C and the β-lactam antibiotics obtained are, if appropriate, freed from the protective groups present and converted into their pharmaceutically usable salts or esters or, if desired, the free acids are prepared from the salts obtained.

5. Process for the preparation of compounds according to Claim 1, characterised in that 3-aminoazetidin-2-ones of the formula IV

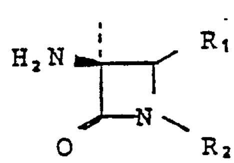

(IV)

in which R, $R_1$ and $R_2$ have the meaning given in Claim 1, are reacted with a derivative, which has acylating action, of a vic-dihydroxyphenylmethyleneamino-(2-oxo-imidazolidine)- or -2,3-dioxo-piperazine)-1-carbonylamino-acetic acid of the formula V

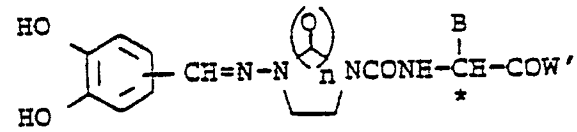

(V)

in which n, B and $\underset{*}{C}$ have the abovementioned meaning and W' is OH or a reactive leaving group, and, if appropriate after any protective groups present have been split off, pharmaceutically usable salts or esters are prepared, or, if desired, the free acids are prepared from the salts obtained.

6. Process for the preparation of compounds according to Claim 1 of the general formula VI,

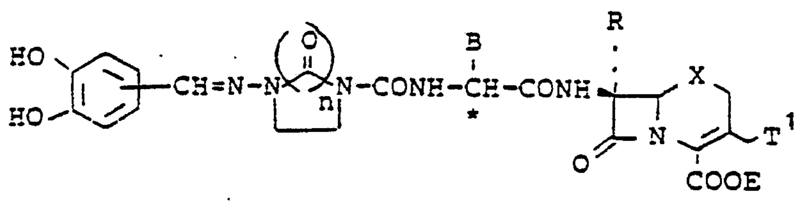

(VI)

in which n, B, $\underset{*}{C}$, R, X and E have the meaning given in Claim 1 and T' has the meaning of T in Claim 1, except hydrogen, characterised in that a compound of the general formula VII

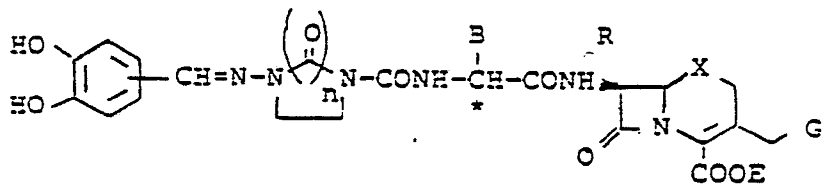

(VII)

in which n, B, $\underset{*}{C}$, R, X and E have the meaning given in Claim 1 and G denotes a nucleofugic group such as halogen, pseudohalogen, acetoxy, dichloroacetoxy or mesyloxy, is reacted with a nucleophile [T'], in which T' has the abovementioned meaning, [G] being eliminated, and, if appropriate after any protective groups have been split off, the products are converted into the pharmaceutically usable salts or esters or, if desired, the free acids are prepared from the salts obtained.

7. Use of the compounds of Claims 1-3 for the preparation of medicaments.

8. Compounds according to Claims 1-3 for use in promoting the growth of animals.

9. Animal feed additives containing compounds according to Claims 1-3.

10. Medicaments containing compounds according to Claims 1-3.

**Revendications**

1. Composés de formule I,

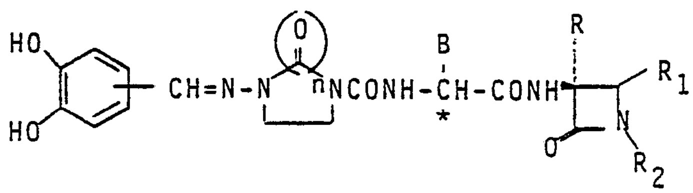

(I)

dans laquelle

B désigne un groupe phényle éventuellement substitué une ou deux fois par un radical méthyle, éthyle, aminométhyle, hydroxy, méthoxy, éthoxy, carbamoyloxy, acétoxy, amino, mésylamino, méthylamino, aminosulfonylamino, guanidyle, carbamoylamino, carboxy, méthoxycarbonyle, carbamoyle, amidino, mésyle, méthylsulfinyle, sulfo, méthylthio ou halogéno ou un groupe cyclohexadiényle, ou un hétérocycle insaturé pentagonal ou hexagonal mono- ou disubstitué par des radicaux méthyle, éthyle, hydroxy, oxy, amino, imino, mésyle, mésylamino, carboxy, carbamoyle, acétyle, ayant 1 à 4 hétéro-atomes

qui peuvent être des atomes d'oxygène, d'azote ou de soufre ;

R désigne l'hydrogène ou le groupe méthoxy ;

n a la valeur 1 ou 2 ;

$R_1$ désigne l'hydrogène ou un groupe alkyle ayant jusqu'à 5 atomes de carbone, qui est éventuellement substitué par un radical hydroxy, amino, carboxy, carbamoyle ou mésyle et qui peut être insaturé ou cyclisé ;

$R_2$ représente $SO_3^{\ominus}M^{\oplus}$ ;

$M^{\oplus}$ désigne un proton ou un cation ;

ou bien $R_1$ et $R_2$ forment conjointement avec le noyau d'azétidinone auquel ils sont liés, un groupe

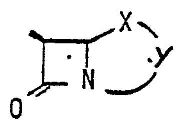

dans lequel

X représente S, O, SO, $SO_2$ ou $CH_2$ ; et

Y représente les groupes

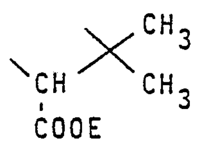

et

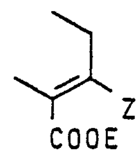

dans lesquels l'atome de carbone qui porte le groupe —COOE est lié à l'atome d'azote du noyau de bêta-lactame et

Z représente l'hydrogène, un halogène, un groupe alkoxy en $C_1$ à $C_3$ ou $—CH_2—T$,

T représente l'hydrogène, les groupes acétate, propionate, n-butyrate et isobutyrate qui peuvent être substitués par des radicaux carboxy, hydroxy ou amino, un groupe pyridinium, carboxamidopyridinium, aminopyridinium, carbamoyloxy, azido, cyano, hydroxy, le groupe —S-phényle qui peut être substitué par un radical méthyle, halogéno, amino, hydroxy ou carboxy, ou le groupe —S-Het dans lequel Het représente un noyau hétérocyclique pentagonal ou hexagonal éventuellement substitué une ou deux fois par un groupe alkyle ayant jusqu'à 3 atomes de carbone, qui peut être substitué par un radical carboxy, sulfo, amino, méthylamino, diméthylamino ou hydroxy, ou par un groupe hydroxy, oxo, amino, imino ou sulfo, ayant 1 à 4 hétéro-atomes qui peuvent être des atomes d'oxygène, d'azote ou de soufre ; et

E désigne l'hydrogène, un groupement ester utilisable pharmaceutiquement, un cation formant un sel ou un groupe protecteur approprié ;

les composés de formule I pouvant exister, relativement au centre de chiralité $\overset{*}{C}$, avec les deux configurations R et S possibles ainsi que comme mélanges des diastéréo-isomères qui en résultent, et les composés de formule I pouvant exister, relativement au groupe imino, tant sous la forme syn que sous la forme anti et ces composés de formule I pouvant aussi exister sous les diverses formes hydratées.

2. Composés suivant la revendication 1, de formule I, dans laquelle

n a la valeur 1 et

$R_2$ désigne un groupe $SO_3^-M^+$ dans lequel $M^+$ désigne un cation alcalin ou alcalino-terreux, le cation aluminium et l'ion ammonium, une amine aliphatique primaire, secondaire ou tertiaire protonée ou une amine hétérocyclique.

3. Composés suivant la revendication 1, dans lesquels

B désigne un groupe phényle qui n'est pas substitué ou qui est substitué une ou deux fois par un radical méthyle, éthyle, aminométhyle, hydroxy, méthoxy, éthoxy, carbamoyloxy, acétoxy, amino, mésylamino, méthylamino, aminosulfonylamino, guanidyle, carbamoylamino, carboxy, méthoxycarbonyle, carbamoyle, amidino, mésyle, méthylsulfinyle, sulfo, méthylthio ou halogéno, ou un noyau furyle, méthylfuryle, thiényle, méthylthiényle, 2-aminothiazolyle, thiazolyle, méthylisoxazolyle, isoxazolyle, pyridyle, 2-aminopyrimidyle, thiadiazolyle, pyrannyle, thiapyrannyle ou sydnonyle, et

T désigne l'hydrogène, les groupes acétate, propionate, n-butyrate et isobutyrate qui peuvent être substitués par un radical carboxy, hydroxy ou amino, un groupe pyridinium, carboxamidopyridinium, aminopyridinium, carbamoyloxy, azido, cyano, hydroxy, le groupe —S-phényle qui peut être substitué par un radical méthyle, halogéno, amino, hydroxy, carboxy, ou le groupe —S-Het dans lequel Het désigne le noyau thiazole, isothiazole, thiadiazolyle, thiazolyle ou tétrazolyle, chacun étant non substitué ou substitué par un radical méthyle, sulfométhyle, carboxyméthyle, diméthylaminoéthyle, le noyau pyridyle, 1-oxydopyridyle, 2-méthyl-5-oxo-6-hydroxy-1,4-dihydro-1,2,4-triazinyle ou le noyau 4-formylméthyl-5-oxo-6-hydroxy-4,5-dihydro-1,2,4-triazinyle.

4. Procédé de production de composés suivant la revendication 1, caractérisé en ce qu'on fait réagir des 3-(amino-acétamido)-azétidine-2-ones de formule III

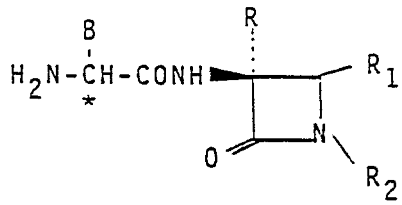

(III)

dans laquelle B, R, $R_1$, $R_2$, $M^{\oplus}$, X, Y, E, Z et T ont les définitions indiquées dans la revendication 1, ces composés de formule III pouvant exister, relativement au centre de chiralité $\overset{*}{C}$, avec les deux configurations R et S possibles ainsi que comme mélanges des diastéréo-isomères qui en résultent, avec des composés de formule générale II

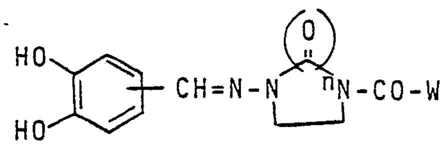

(II)

dans laquelle

W représente un halogène, un groupe azide ou un autre groupe partant nucléofuge,

n a la valeur 1 ou 2, et les deux groupes hydroxy peuvent être protégés par des groupes silyle ou cyclosilyle,

en présence d'un solvant et, le cas échéant, d'un accepteur d'acide à des températures d'environ — 20 à environ + 50 °C, et les antibiotiques de type bêta-lactame obtenus sont éventuellement débarrassés des groupes protecteurs présents et transformés en leurs sels ou esters utilisables du point de vue pharmaceutique, ou bien si on le désire, les acides libres sont préparés à partir des sels obtenus.

5. Procédé de production de composés suivant la revendication 1, caractérisé en ce qu'on fait réagir des 3-aminoazétidine-2-ones de formule IV

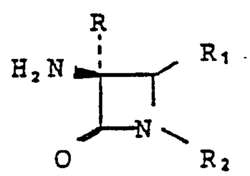

(IV)

dans laquelle R, $R_1$ et $R_2$ ont la définition indiquée dans la revendication 1 avec un dérivé à action acylante d'un acide vic-dihydroxy-phénylméthylène-amino-(2-oxo-imidazolidine)- ou -(2,3-dioxo-pipérazine)-1-carbonylaminoacétique de formule V

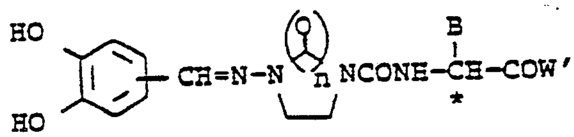

(V)

dans laquelle n, B et $\underset{*}{C}$ ont la définition indiquée ci-dessus et W' est un groupe OH ou un groupe partant réactif et, le cas échéant, après élimination des groupes protecteurs présents, on prépare des sels ou esters utilisables pharmaceutiquement ou on prépare, lorsqu'on le désire, les acides libres à partir des sels obtenus.

6. Procédé de production de composés suivant la revendication 1, de formule générale VI

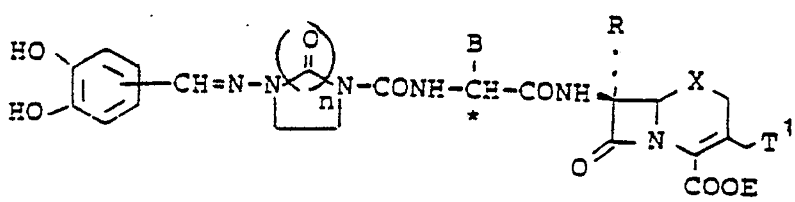

(VI)

dans laquelle n, B, $\underset{*}{C}$, R, X et E ont la définition indiquée dans la revendication 1 et T' a la définition de T dans la revendication 1, excepté l'hydrogène, caractérisé en ce qu'on met en réaction un composé de formule générale VII

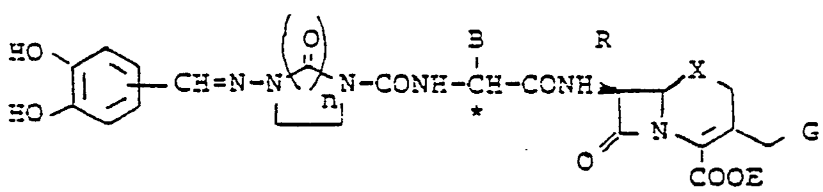

(VII)

dans laquelle n, B, C, R, X et E ont la définition indiquée dans la revendication 1 et G est un groupe nucléofuge tel qu'halogéno, pseudohalogéno, acétoxy, dichloracétoxy, mésyloxy, avec un nucléophile [T'], où T' a la définition indiquée ci-dessus, avec départ de [G] et, le cas échéant, après élimination des groupes protecteurs présents, on les transforme en sels ou esters utilisables du point de vue pharmaceutique ou bien on prépare, si on le désire, les acides libres à partir des sels obtenus.

7. Utilisation des composés des revendications 1 à 3 pour l'obtention de médicaments.

8. Composés suivant les revendications 1 à 3, destinés à être utilisés pour activer la croissance d'animaux.

9. Additifs pour l'alimentation des animaux, contenant des composés suivant les revendications 1-3.

10. Médicament contenant des composés suivant les revendications 1-3.